# EUROPEAN PATENT SPECIFICATION

(11) **EP 4 121 073 B1**
(45) Date of publication and mention of the grant of the patent: **03.07.2024**
(21) Application number: 21710991.7
(22) Date of filing: 15.03.2021
(51) Int. Cl.: A61K 35/741, A61K 31/715, A61K 35/745, A61K 35/747, A61K 38/39, A61L 27/24, A61P 15/02, A61K 9/00

(54) **BIOCOMPOSITES COMPRISING PROBIOTICS, COLLAGEN AND BACTERIAL EXTRACELLULAR POLYSACCHARIDE AND USES THEREOF**
BIOKOMPOSITE MIT PROBIOTIKA, KOLLAGEN UND BAKTERIELLEM EXTRAZELLULÄREM POLYSACCHARID UND VERWENDUNGEN DAVON
BIOCOMPOSITES COMPRENANT DES PROBIOTIQUES, DU COLLAGÈNE ET UN POLYSACCHARIDE EXTRACELLULAIRE BACTÉRIEN ET LEURS UTILISATIONS

(30) Priority: 16.03.2020 EP 20382195
(43) Date of publication of application: 25.01.2023
(73) Proprietor: Biosearch S.A., 18004 Granada (ES)
(72) Inventor: OLIVARES MARTÍN, Mónica, 18004 Granada (ES); BAÑUELOS HORTIGÜELA, Óscar, 18004 Granada (ES); DOMÍNGUEZ VERA, José Manuel, 18100 Armilla, Granada (ES); GONZÁLEZ GARNICA, Ana, 18003 Granada (ES); DELGADO LÓPEZ, José Manuel, 18100 Armilla, Granada (ES); SABIO RODRÍGUEZ, Laura, 18008 Granada (ES); RAMÍREZ RODRÍGUEZ, Gloria Belén, 14510 Moriles, Córdoba (ES)
(74) Representative: FRKelly
(86) International application number: PCT/EP2021/056471
(87) International publication number: WO 2021/185728

(56) References cited:
- WO-A1-2014/184643
- WO-A1-2020/018504
- ANA GONZÁLEZ ET AL: "Entrapping Living Probiotics into Collagen Scaffolds: A New Class of Biomaterials for Antibiotic-Free Therapy of Bacterial Vaginosis", ADVANCED MATERIALS TECHNOLOGIES, vol. 5, no. 7, 25 May 2020 (2020-05-25), page 2000137, XP055722405, DE ISSN: 2365-709X, DOI: 10.1002/admt.202000137
- RAN SU ET AL: "Encapsulation of probioticBIOMA 5920 with alginatehuman-like collagen and evaluation of survival in simulated gastrointestinal conditions", INTERNATIONAL JOURNAL OF BIOLOGICAL MACROMOLECULES, ELSEVIER BV, NL, vol. 49, no. 5, 19 August 2011 (2011-08-19), pages 979-984, XP028321965, ISSN: 0141-8130, DOI: 10.1016/J.IJBIOMAC.2011.08.018 [retrieved on 2011-08-26]
- MICHAEL T COOK ET AL: "Microencapsulation of probiotics for gastrointestinal delivery", JOURNAL OF CONTROLLED RELEASE, ELSEVIER, vol. 162, no. 1, 3 June 2012 (2012-06-03), pages 56-67, XP028411618, ISSN: 0168-3659, DOI: 10.1016/J.JCONREL.2012.06.003 [retrieved on 2012-06-11]

## Description

### FIELD OF THE INVENTION

The invention relates to the field of therapeutics and, more particularly, to the therapy of bacterial vaginosis using probiotic compositions which are provided forming part of a biological biocomposite material.

### BACKGROUND OF THE INVENTION

Bacterial vaginosis (BV) is the most frequent cause of vaginal disorders in women of reproductive age. The most common symptoms of this infection are the dense abnormal vaginal discharge, pain, itching and an unpleasant odor. Independent studies have validated that the BV condition increases the risk of developing other pathologies, such as pelvic inflammatory disease and sexually transmitted infections (STIs), including the human immunodeficiency virus (HIV). In addition, BV has been strongly associated with premature birth and postpartum uterine infections.

From a microbiology perspective, BV is characterized by the alteration of the normal balance of the vaginal microbiota, and the pathological condition typically referred as 'vaginal dysbiosis'. The native microbiota helps to create a protective barrier for vaginal mucosa against infections. However, the healthy microbiota is sensitive to several factors, in particular, a change in the pH of the mucosa, which can unbalance the microbial populations, favoring the appearance of infection- causing microbes. The health of the vaginal microbiota is believed to be maintained by lactic acid- producing organisms, such as Lactobacilli.] With a minute imbalance in the pH profile of the vaginal fluid (e.g. from pH 4 to 5 in a healthy vs. unhealthy environment), these Lactobacilli are suppressed by the overgrowth of pathogenic bacteria, such as *Gardnerella* vaginalis and *Mobiluncus* spp., which are present in small populations when healthy, resulting in BV in reproductive-age women.

From a chemical point of view, the vaginal environment of a healthy woman is dominated by the lactic acid, the main metabolite of the Lactobacillus family. Other short-chain fatty acids (SCFAs) such as acetic, propionic and butyric acids associated with other typically harmful bacteria are present at almost 100-fold lower concentrations. The pH is therefore regulated by the lactic acid (pKa = 3.80), which is found in healthy vaginal fluid at concentrations of the order of 120 mM, leading pH values around 4. When BV develops, the decreasing population of Lactobacilli and the excessive growth of pathogenic bacteria modify the metabolite pattern: an increase of SCFAs and a dramatic decrease of lactic acid up to concentrations around 20 mM. In particular, acetic acid, the main component of SCFAs, can reach concentrations about 120 mM from originally 2 mM, which leads to an increase of pH up to the value of 5, since the pKa of acetic acid (4.86) is higher than that of lactic acid. Therefore, the pH of the vaginal fluid moves from 4 (healthy) to 5 (BV) and worsening the disease condition over time. The pH change is, in fact, a key parameter used in the diagnosis of BV. For instance, the Amsel method diagnoses BV when the vaginal fluid has a pH higher than 4.5.

BV has been traditionally treated with antibiotics, especially metronidazole. However, a number of drawbacks in existing treatment modalities necessitate the development of new routes of therapy. Firstly, treatments based on antibiotics do not avoid recurrences. Secondly, the development of antibiotic-resistant bacteria due to the excessive use of antibiotics remains the biggest challenge faced by modern medicine. Therefore, more effective long-term therapies that reduce our reliance on antibiotics are critically needed.

In view of the biochemical cycle taking place during BV, i.e., the decrease of lactic acid concentration that ultimately facilitates the overgrowth of pathogens, one of the potential treatment strategies against BV may be to directly apply lactic acid to the infected vagina. While this treatment may reduce the symptoms, frequent and prolonged use of this therapy is expected to avoid the reappearance of BV. More recent approaches have considered the use of probiotics of the Lactobacillus family. For instance, WO2014184643 describes compositions having antibacterial activity against bacteria causing vaginosis and which contain probiotics of the Lactobacillus family. While promising, the efficiency of this approach will depend on the adhesion and proliferation levels of the probiotic within the BV-compromised microproliferation and subsequent therapy, leading to BV recurrence.

### SUMMARY OF THE INVENTION

The authors of the present invention have developed a new strategy for the treatment of BV based on the use of a biocompatible collagen-based matrix that protects probiotics and allows their effective localization and proliferation within the compromised vaginal fluids. In particular, the inventors have shown that collagen scaffolds (col) are capable of protecting two biofilm-producing probiotics of the genus lactobacillus, viz. *L. fermentum* (*Lf*) and *L. acidophilus* (*La*), resulting in hybrid materials, col-*Lf* and col-La, respectively, with enhanced healing properties against BV infections. Both materials correspond to biocomposites composed of collagen, probiotic and the bacterial EPS (exopolysaccharides). col-*Lf* and col-*La* are able to restore the pH of a compromised simulated BV fluid to a healthy condition. Outstanding stability of these collagen-entrapped probiotics, combined with high adhesion to the vaginal mucosa and the intrinsic biocompatibility of the collagen matrix, make these materials very promising for non-antibiotic BV therapy

### BRIEF DESCRIPTION OF THE DRAWINGS

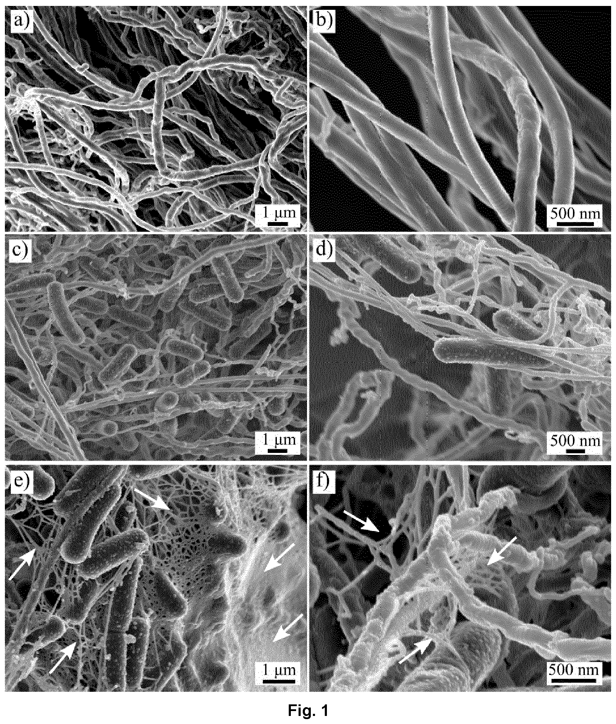

**Figure 1****.** SEM micrographs of collagen fibers assembled in PBS (pH 7.4) in the absence (a-b) and presence of the probiotic *Lf* (c-d) and then, incubated in MRS to produce col-*Lf* (e-f). Arrows indicate the EPS.

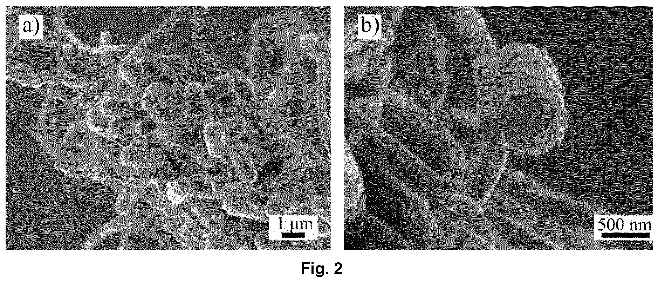

**Figure 2****.** SEM micrographs of collagen fibers in the presence of the probiotic La. Different magnifications: 26.60 kx (a) and 95.63 kx (b).

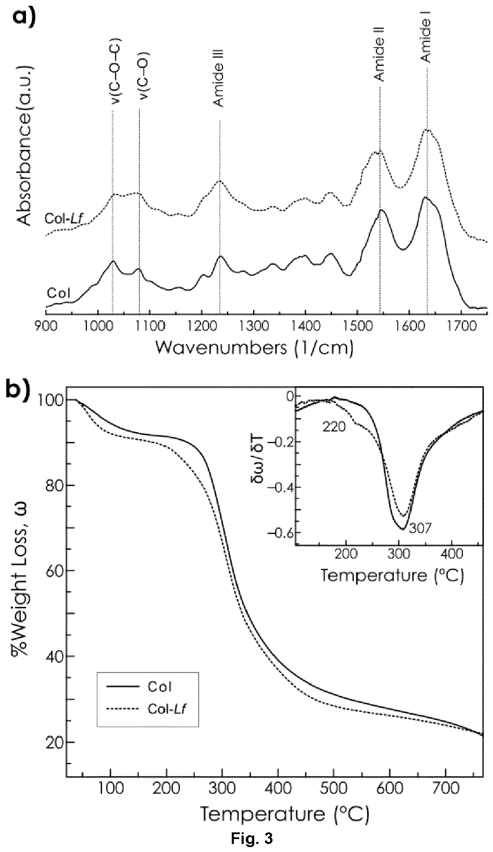

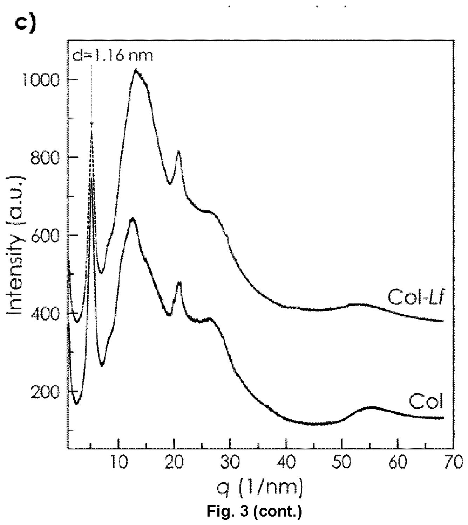

**Figure 3****:** FTIR spectra (a) and TGA curves (b) of collagen (Col) and col-*Lf*. The inset in (b) shows the corresponding derivative TGA curves (DTG curves). Both characterizations confirm the effective incorporation of the bacteria into the collagen scaffolds. (c) X-ray diffraction patterns of col and col-*Lf*. *q* represents the scattering vector amplitude, *q* = 4πsinθ/λ, being θ the scattering angle and λ the wavelength of the X-ray beam.

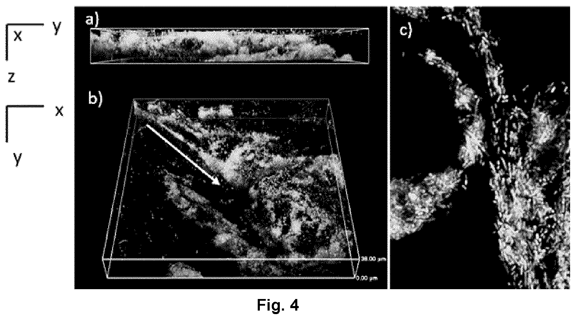

**Figure 4****.** Confocal images of col-*Lf* stained with the SYTO9 (green) and propidium iodide (red) dyes. a) yz-section reveals that the bacteria penetrated and are fully integrated into the collagen matrix, b) 3D image (318.20 × 318.20 × 38 µm³) showing how bacteria are specifically aligned along the collagen fibers (arrow). c) A higher magnification image showing very few dead (red) bacteria (this image is a maximum intensity projection of 6 microns in depth).

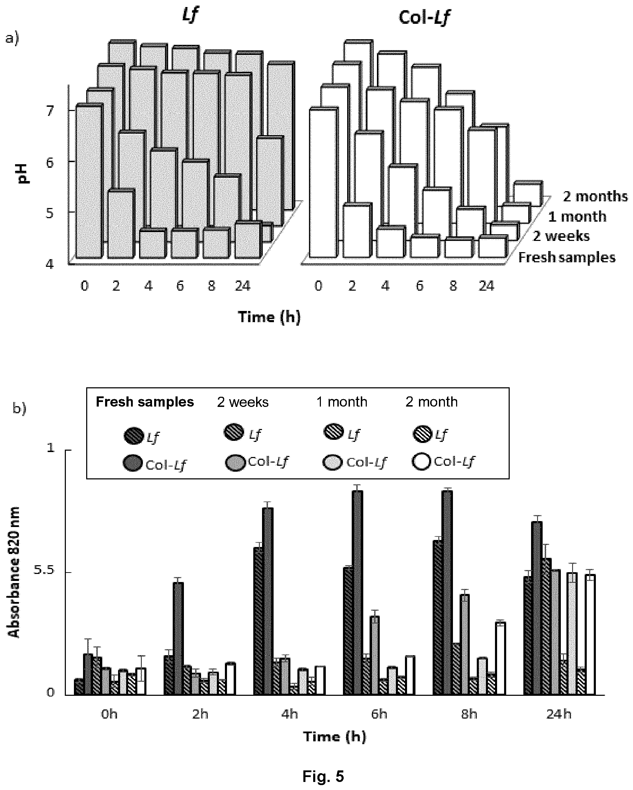

**Figure 5****.** a) Time evolution of the pH in 1/10 diluted MRS media containing *Lf* or col-*Lf* after storage for two, four and eight weeks at 4 °C. The pH evolution of fresh samples is also shown. Errors as standard deviations for these values were less than ± 0.2. b) Time evolution of the POM reduction activity in 1/10 diluted MRS containing *Lf* or col-*Lf* after storage for two weeks, 1 month or 2 months at 4 °C. Evolution of the POM reduction activity of fresh samples is also shown. Error bars show standard deviations.

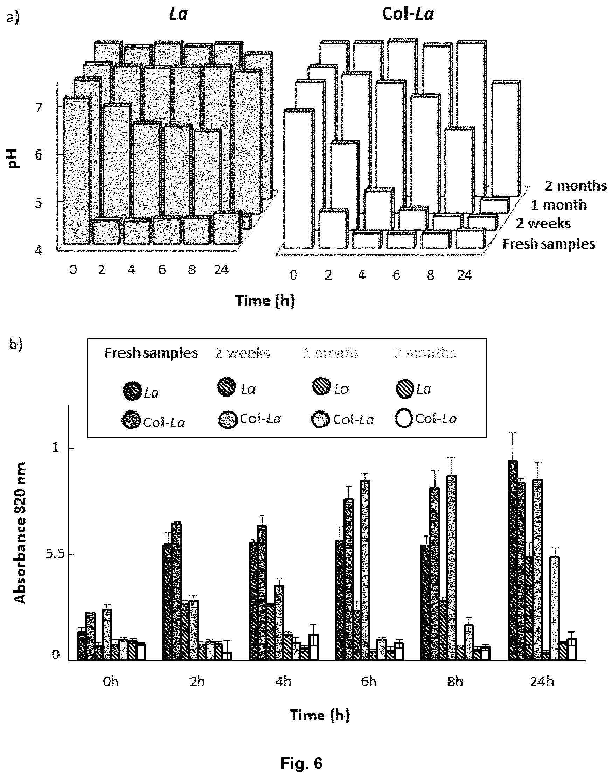

**Figure 6****:** a) Time evolution of the pH in 1/10 diluted MRS media containing *La* or col-*La* after storage for two weeks, 1 month or 2 months at 4 °C. Evolution of the pH of fresh samples is also shown. Errors as standard deviations for these samples are all less than ± 0.2. b) Time evolution of the POM reduction activity in 1/10 diluted MRS containing La or col-*La* after storage for two weeks, 1 month or 2 months at 4 °C. Evolution of the POM reduction activity of fresh samples is also shown. Error bars show standard deviations.

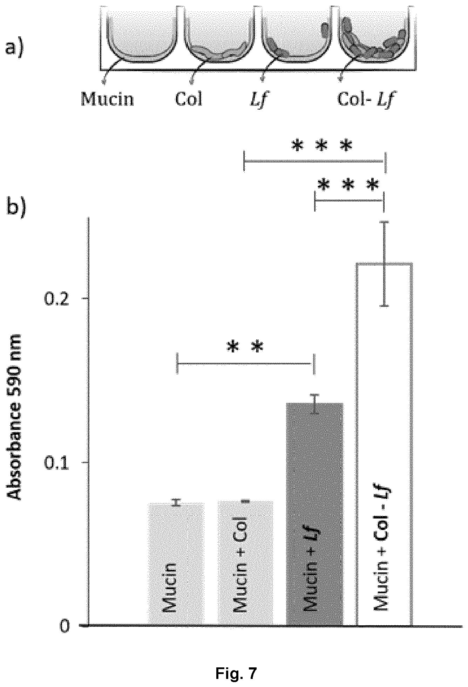

**Figure 7**. a) Schematic representation of the adhesion experiments. b) Evaluation of the adhesion of *Lf* and col-*Lf* to immobilized mucin. Absorbance at 590 nm correlates with the amount of bacteria adhered to mucin. Absorbance values of controls (Mucin and Mucin + col) are also included. Data are expressed as average ± SD (n=3). ** p < 0.01 and *** p <0.005.

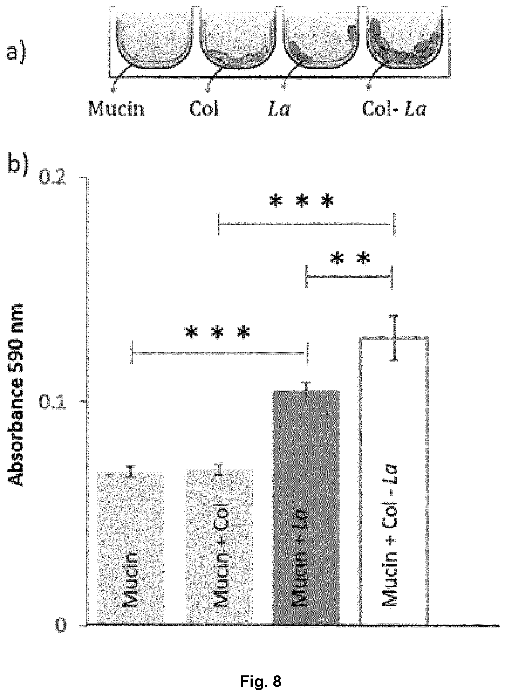

**Figure 8****:** a) Schematic representation of the adhesion evaluation experiment. b) Evaluation of the adhesion of *La* and col-*La* to immobilized mucin. Absorbance at 590 nm correlates with the amount of bacteria adhered to mucin. Absorbance values of controls (Mucin and Mucin + col) are also included. Data are expressed as average ± SD (n=3). ** p < 0.01 and *** p < 0.005.

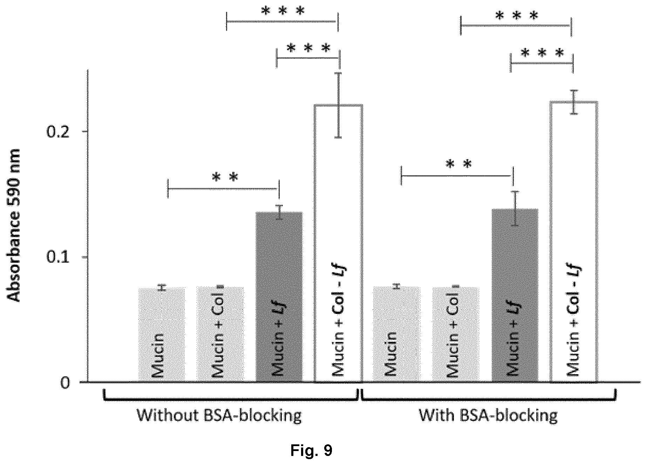

**Figure 9****:** Evaluation of the adhesion of *Lf* and col-Lf to immobilized porcine mucin. Absorbance at 590 nm correlates with the amount of bacteria adhered to mucin. Absorbance values at 590 nm of controls (Mucin and Mucin + col) are also included. Results while blocking or not with BSA are shown. Error bars show standard deviations.

Asterisk indicates a significant difference (** is p < 0.01 and *** is p < 0.005) in the adhesion to mucin.

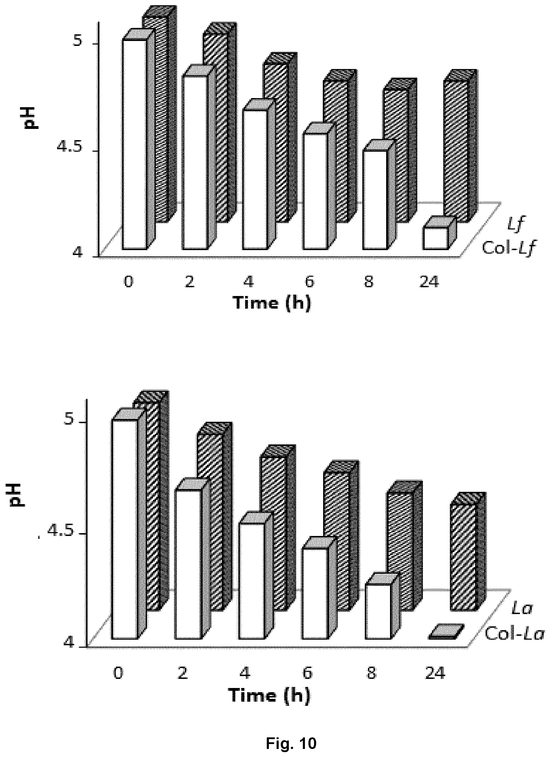

**Figure 10****.** pH evolution of col-*Lf* and col-*La*, *Lf* and *La* in a simulated BV fluid (initial pH 5.0). Errors as standard deviations were all less than ± 0.06.

### DETAILED DESCRIPTION

### Method for obtaining a biocomposite

In a first aspect, the invention relates to a method for preparing a biomaterial comprising collagen, probiotics, and exopolysaccharide wherein the method comprises:
i) contacting a population of probiotic bacteria with collagen monomers,
ii) incubating the mixture obtained in step i) under conditions adequate for the and
iii) maintaining the collagen fibers containing entrapped probiotic bacteria obtained in step ii) under conditions adequate for the formation of exopolysaccharide (EPS) by the probiotics until the content of EPS in the biomaterial with respect to the collagen content of the biomaterial measured as the sugar content is of at least 2% (w/w).

In a first step, the method according to the invention comprises contacting a population of probiotic bacteria with collagen monomers.

The term "population of probiotic bacteria" or "probiotic composition", as used herein, refers to a mono or mixed culture of microorganisms which when provided to a mammal, for example a human, affect the host beneficially. In some embodiments, a probiotic composition of the invention contains a single species of bacteria. In other embodiments, the probiotic composition contains two or more species of bacteria, e.g., 2, 3, 4, 5, 6, 7, 8, 9, 10, 15, 20, 30, 40, 50, 60, 70, 80, 90, 100, 500, 1000 or more species of bacteria. In one embodiment, the probiotic composition contains no more than 20 species of bacteria, e.g., 20, 19, 18, 17, 16, 15, 14, 13, 12, 11, 10, 9, 8, 7, 6, 5, 4, 3, 2, or 1 species of bacteria. In exemplary embodiments, the probiotic composition contains 8 bacterial species. In other exemplary embodiments, the probiotic composition contains 9 bacterial species. In other embodiments, the probiotic composition contains or is administered in conjunction with a prebiotic, as described herein.

In one embodiment, the population of probiotic bacteria contains at least one strain which is capable of producing biofilm.

The term "biofilm", as used herein, refers to an extracellular matrix in which microorganisms are dispersed and/or form colonies. The biofilm typically is made of polysaccharides and other macromolecules. Strains capable of producing biofilms that can be used in the present invention can be identified by using an assay as described in example 1, wherein the strain is grown within a collagen scaffold in a compatible medium scaffold and the increase in the sugar content is determined. As explained below, strains which are capable of forming biofilm are defined as those which lead to a sugar content in the final material of at least 2,5% (w/w), at least 3%, at least 3,5%, at least 4%, at least 4,5%, at least 5%, at least 5,5%, at least 6%, at least 6,5%, at least 7%, at least 7,5%, at least 8%, at least 8,5%, at least 9%, at least 9,5%, at least 10%, at least 11%, at least 12%, at least 13%, at least 14%, at least 15%, at least 16%, at least 17%, at least 18%, at least 19%, at least 20%, at least 25%, at least 30%, at least 35%, at least 40%, at least 50% or more, the values being given in w/w with respect to the collagen content in the biocomposite. It will be understood that the Strains capable of producing biofilms can also be identified using SEM by detecting the formation of a network in the biomaterial that corresponds to the EPS.

Preferred bacterial genera to be used as probiotic compositions in the method of the invention include *Lactobacillus* and *Bifidobacterium.*

The term "lactic acid bacterium" or "LAB", as used herein, refers to any bacterium capable of producing, as the major metabolic end product of carbohydrate fermentation, lactic acid or at least one of its derivatives (including, but not limited to, acetic acid or propionic acid): the term is therefore intended to include propionic acid bacteria (PAB), which produce propionic acid as a carbohydrate fermentation product.. Illustrative non-limitative examples of lactic acid bacteria are bacteria of the genera *Lactobacillus*, *Leuconostoc*, *Pediococcus*, *Lactococcus*, *Propionibacterium* and *Streptococcus* as well as the genera of the order *Lactobacillales Aerococcus*, *Carnobacterium*, *Enterococcus*, *Oenococcus*, *Teragenococcus*, *Vagococcus* and *Weisella.* Typically, the lactic acid bacterium is selected from the species *Leuconostoc spp.*, *Lactococcus cremoris*, *Lactococcus lactis*, *Lactobacillus acidophilus, Lactobacillus casei*, *Lactobacillus kefiri, Lactobacillus bifidus*, *Lactobacillus brevis*, *Lactobacillus helveticus*, Lactobacillus paracasei, Lactobacillus rhamnosus, *Lactobacillus salivarius*, *Lactobacillus curvatus*, Lactobacillus bulgaricus, Lactobacillus sakei, *Lactobacillus reuteri*, *Lactobacillus fermentum, Lactobacillus farciminis, Lactobacillus lactis*, *Lactobacillus delbreuckii, Lactobacillus plantarum, Lactobacillus paraplantarum, Lactobacillus crispatus, Lactobacillus gasseri*, *Lactobacillus johnsonii* and *Lactobacillus jensenii.*

In a preferred embodiment, the lactic acid bacterium is a bacterium of the genus Lactobacillus. The term "Lactobacillus", as used herein, refers to a genus which is described in the NCBI database by the Taxonomy ID 1578. In a particular embodiment, the lactic acid bacterium is selected from the group consisting of *Lactobacillus fermemtun, Lactobacillus gasseri, Lactobacillus reuteri, Lactobacillus coryniformis*, *Lactobacillus casei*, *Lactobacillus paracasei, Lactobacillus plantarum, Lactobacillus salivarius* and *Lactobacillus bulgaricus.* In a more preferred embodiment, the lactic acid bacterium is *Lactobacillus fermentum.* The term "*Lactobacillus fermentum*" or "L. *fermentum*" refers to a species of the genus Lactobacillus which is described in the NCBI database by the Taxonomy ID 1613.

Exemplary strains of L. gasseri include those deposited as having American Type Culture Collection (ATCC) accession number (ATCC No.) 33323, ATCC No. 19992, ATCC No. 4484, ATCC No. 4479, ATCC No. 4481, ATCC No. 4483, ATCC No. 4480, ATCC No. 4962, ATCC No. 9857, ATCC No. 4963, ATCC No. 29601, ATCC No. 33323. One exemplary strain of L. gasseri is L. gasseri JPS. Lactobacillus casei is a species of genus Lactobacillus widely used for dairy product fermentation. Exemplary strains of L. casei include a strain having ATCC accession number PTA-3945, which can also be referred to as L. casei KE01 or L. casei KE99. L. casei PTA-3945 is described in U.S. Pat. No. 6,797,266. Additional exemplary strains include ATCC No. 393, ATCC No. 334, ATCC No. 4007, ATCC No. 39539, ATCC No. 27139, ATCC No. 4940, ATCC No. 39392, ATCC No. 4913, ATCC No. 4646, ATCC No. 15008, ATCC No. 334D-5, ATCC No. PTA-2662, ATCC No. 55825, ATCC No. 55826, ATCC No. 55841, ATCC No. PTA-5149, ATCC No. 7469, ATCC No. 25598, ATCC No. 27216, ATCC No. 25599, ATCC No. 25302, ATCC No. 11582, ATCC No. 11982, ATCC No. 39595, ATCC No. 25180, ATCC No. 11578, ATCC No. 9595, ATCC No. 14435, ATCC No. 12116, ATCC No. 25303, ATCC No. 13075, ATCC No. 29599, ATCC No. 27092, ATCC No. 11981, ATCC No. 27773, ATCC No. 8530, ATCC No. 49178, ATCC No. 335, ATCC No. 14957, ATCC No. 7469a, ATCC No. HB-12558, ATCC No. HB-12560, ATCC No. HB-12559, ATCC No. 15820, ATCC No. 27792, ATCC No. 25937, ATCC No. 27092-B1, ATCC No. 87074. Lactobacillus acidophilus is a species of genus Lactobacillus that occurs naturally in the gastrointestinal tract of humans and animals and is a part of normal vaginal flora. Some strains of Lactobacillus acidophilus are used in dairy production. Exemplary Lactobacillus acidophilus strains are ATCC No. 4356, ATCC No. 3154, ATCC No. 53544, ATCC No. 53544, ATCC No. 43121, ATCC No. PTA-4482, ATCC No. 4796, ATCC No. 53546, ATCC No. 4355, ATCC No. 9224, ATCC No. 4357, ATCC No. 832, ATCC No. 4357D-5, ATCC No. PTA-6751, ATCC No. 6820.

In a preferred embodiment, the probiotic is *L. fermentum* and/or *L. acidophilus.* In a still more particular embodiment, the probiotic forming part of the probiotic composition is *L*. *fermentum* CECT5716, *L. acidophilus* CECT903 or a combination of both.

*L. fermentum* CECT5716 is a strain isolated from human breast milk which has been disclosed in PCT application published as WO2004003235.

*L. acidophilus* CECT903 is a strain isolated from human and which is freely available from Colección Española de Cultivos Tipo (https://www.cect.org/vstrn.php?lan=es&cect=903).

As provided herein, therapeutic compositions comprise, or in the alternative, modulate, the colonization and/or engraftment, of the following exemplary bacterial entities: *Lactobacillus gasseri, Lactobacillus fermentum, Lactobacillus reuteri, Lactobacillus acidophilus, Lactobacillus plantarum, L. crispatus, L. jensenii* and,. *B. breve*, *B. longum, B. bifidum, B. dentium.*

The term "bacterium of the genus Bifidobacterium" or "bifidobacterium", as used herein, refers to a genus of bacteria which is described in the NCBI database with the Tanoxonomy ID 1678. Illustrative non- limitative examples of suitable Bifidobacteria are the species *Bifidobacterium lactis*, *Bifidobacterium bifidium*, *Bifidobacterium longum*, *Bifidobacterium animalis, Bifidobacterium breve, Bifidobacterium infantis, Bifidobacterium catenulatum, Bifidobacterium pseudocatenulatum, Bifidobacterium adolescentis*, and *Bifidobacterium angulatum.* In a particular embodiment, the bacterium of the genus Bifidobacterium is selected from the group consisting of *Bifidobacterium breve, Bifidobacterium longum*, *Bifidobacterium animalis*, *Bifidobacterium infantum* and *Bifidobacterium animalis.*

In one embodiment, the probiotic composition comprises a single probiotic species. In other embodiment, when the probiotic composition comprises more than one probiotic species, the probiotic composition does not comprise the strain of bacteria selected from the group comprising the strain *Lactobacillus plantarum* LMG P-21021-LP01, the strain *Lactobacillus plantarum* LMG P-21020 -LP02, the strain *Lactobacillus fermentum* DSM 26955 (LF15) or the strain *Lactobacillus fermentum* DSM 26956 (LF16).

The term collagen, as used herein, refers to a self-assembling biopolymer produced by reacting monomeric collagen in a neutral pH, slightly ionic solution.

The predominantly monomeric solution is suitably aqueous or non-aqueous, depending on the monomer. In preferred embodiments, the pH of the solution is controlled, in specific preferred embodiments, the solution is an aqueous solution, and the pH is buffered, preferably in the range of about 7.2 to about 7.6. Any buffer capable of maintaining the pH within this range is suitable; a preferred buffer is a phosphate buffer. The predominantly monomeric solution can include one or more additives besides the monomers. Additives can be chosen to roles such as promoting polymerization, combining with the monomers to form a copolymer, or providing a coating on the polymer structures. In embodiments in which the polymer is collagen, a preferred additive is one or more glycosaminoglycans selected from the group consisting of hyaluronan, chondroitin sulfate, dermatan sulfate, keratin sulfate, or proteoglycans selected from the group including decorin, lumican, biglycan, keratocan, syndican and mixtures thereof. Additives can be chosen to modify the physical properties of the monomer solution. For example, glycerol can be added to adjust the viscosity of the monomer solution. In other embodiments, a surfactant can be added to improve wetting of the substrate.

The collagen construct can be an unoriented collagen gel, a tissue-derived collagen template or a nanostructured artificial template. Typically, the collagen construct comprises a collagen is selected from the group consisting of Type I collagen, Type N collagen, and mixtures thereof, preferred embodiments, the collagen construct comprises Type I collagen. In other embodiments, the collagen construct comprises a mixture of Type I collagen and Type N collagen. Preferably, the construct comprises more Type I collagen than Type N collagen, in one preferred embodiment, the construct comprises a mixture of about four parts Type I collagen to about one part Type N collagen, in some embodiments, the collagen construct can also include a collagen that is selected from the group consisting of Type II collagen, Type III collagen, Type XI collagen, Type IV collagen, and mixtures thereof. The self-assembled collagen fibrils can be homotypic or heterotypic. In one embodiment, the collagen is type I collagen.

The collagen fibril matrix building blocks used to construct the collagen compositions described herein can be obtained from a number of sources including, for example, porcine skin. Suitable tissues useful as a collagen-containing source material for isolating collagen or collagen components for making the collagen compositions described herein are warm-blooded vertebrate submucosa or any other extracellular matrix-containing tissue. Suitable methods for preparing submucosa are disclosed in US Pat. Nos. 4,902,508; 5,281,422. And in US Pat. No. 5,275,826. Tissues containing extracellular matrix material other than submucosal tissue can be used to obtain collagen according to yet other embodiments disclosed herein. Methods for preparing tissue from other extracellular matrix material for use in obtaining purified collagen or partially purified extracellular matrix components will be known to those skilled in the art. For example, US Pat. No. 5,163,955 (pericardial tissue); US Pat. No. 5,554,389 (bladder submucosa); No. 099,567 (gastric submucosa); 6,576,265 (generally extracellular matrix tissue); 6,793,939 (liver basement membrane tissue); 919, 121 (basement tissue of liver); and WO 2001/45765 (generally extracellular matrix tissue). In various other embodiments, the collagen-containing source material can be selected from the group consisting of placental tissue, ovarian tissue, uterine tissue, animal tail tissue, skin tissue, bone, tendon and cartilage tissue. In some embodiments, the collagen is selected from porcine skin collagen, bovine collagen and human collagen; however, any suitable extracellular matrix-containing tissue is purified collagen or partially purified extracellular according to the present teachings. It will be understood and appreciated herein that it can be used as a collagen-containing source material for isolating matrix components. In one embodiment, the collagen is type I collagen isolated from equine tendons.

As mentioned above, one building block used to prepare a collagen fibril matrix is oligomeric collagen. The presence of oligomeric collagen allows the self-assembly of building blocks into the collagen fibril matrix, increases the rate of assembly, collagen composition with clear fibril microstructure and excellent mechanical integrity (e.g. stiffness).

In some embodiments, the building block for the collagen fibril matrix also includes various proportions of non-oligomeric soluble collagen molecules. In one embodiment, the building block comprises one or both of telocollagen and/or atelocollagen. In certain embodiments, the building block comprises oligomeric collagen and atelocollagen. In other embodiments, the building block comprises oligomeric collagen and telocollagen. In yet other embodiments, the building block comprises oligomeric collagen, telocollagen, and atelocollagen. The amount of oligomeric collagen, telocollagen, atelocollagen and / or other non-oligomeric soluble collagen molecules can result from synthetic collagen fibrils resulting from, for example, stiffness, strength, fluid and mass transport, proteolysis and / or compatibility. It can be formulated in solution prior to the start of polymerization to adjust one or more properties of the matrix. It will be appreciated that the predetermined ratio of oligomeric to non-oligomeric soluble collagen molecules for use with a particular active agent may be different from that suitable for use with a different active agent.

Collagen concentration can be expressed in mass / volume or mass / mass. Collagen content can be measured by any means known in the art including, but not limited to, calibrated colorimetric assays such as amino acid analysis for Sirius red and hydroxyproline. The viscosity of a collagen polymer formulation is affected by several factors including, but not limited to, solution or dispersion/suspension, concentration, molecular composition, molecular size, temperature and operating conditions. Viscosity measurements may be obtained by any means known in the art, including but not limited to viscometers or rheometers.

The concentration of soluble collagen present in the composition in step (i) of the invention can vary. In some embodiments, the collagen is present at a concentration of about 0.5 mg/ml to about 500 mg / ml. In other embodiments, the collagen is present at a concentration of about 0.05 mg/ml to about 100 mg/ml. In yet other embodiments, the collagen is present at a concentration of about 0.1 mg/ml to about 100 mg/ml. In some embodiments, the collagen is present at a concentration of about 0.2 mg/ml to about 50 mg/ml. In other embodiments, the collagen is present at a concentration of about 0.3 mg/ml to about 40 mg/ml. In yet other embodiments, the collagen is present at a concentration of about 0.4 mg/ml to about 30 mg/ml. In yet other embodiments, the collagen is present at a concentration of about 0,5 mg/ml to about 20 mg/ml. In some embodiments, the collagen is present at a concentration of about 0.6 mg/ml to about 10 mg/ml. In some embodiments, the collagen is present at a concentration of about 0.7 mg/ml to about 8 mg/ml. In some embodiments, the collagen is present at a concentration of about 0.8 mg/ml to about 6 mg/ml. In some embodiments, the collagen is present at a concentration of about 0.9 mg/ml to about 4 mg/ml. In some embodiments, the collagen is present at a concentration of about 1 mg/ml to about 8 mg/ml. In some embodiments, the collagen is present at a concentration of about 1.1 mg/ml to about 2 mg/ml. In some embodiments, the collagen is present at a concentration of about 1.2 mg/ml to about 1.8 mg/ml. In some embodiments, the collagen is present at a concentration of about 1.3 mg/ml to about 1,7 mg/ml. In some embodiments, the collagen is present at a concentration of about 1.4 mg/ml to about 1.6 mg/ml. In some embodiments, the collagen is present at a concentration of about 1.5 mg/ml.

The pH the monomeric collagen solution used in step (i) is not particularly limiting as long as it is acidic enough so as to prevent the collagen for spontaneously associating into fibrils prior to step (ii) or prior to the contacting with the probiotic. In one embodiment, the pH of the monomeric collagen solution is of about 1 to 4. In one embodiment, the pH of the monomeric collagen solution is of about 1.5 to 3.5. In one embodiment, the pH of the monomeric collagen solution is of about 2 to 3. In one embodiment, the pH of the monomeric collagen solution is of about 2.4 to 2.8. In one embodiment, the pH of the monomeric collagen solution is of about 3 to 4. In one embodiment, the pH of the monomeric collagen solution is of about 3.0.

In the embodiments described herein, the synthetic collagen fibril matrix may have an oligomer content that is quantified by average polymer molecular weight (AMW). As described herein, modulation of AMW is dependent on matrix polymerization kinetics, fibril microstructure, molecular properties, and fibril structure, such as interfibril branching, pore size, and mechanical integrity (e.g. Matrix stiffness). In another embodiment, the oligomer content of the prematrix composition quantified by the average polymer molecular weight is positively correlated with the matrix firmness.

In some embodiments, the non-oligomeric soluble collagen included in the composition used in step (i) is reduced collagen. As used herein, "reduced collagen" means collagen that has been reduced in vitro to remove or substantially reduce reactive aldehydes. For example, collagen can be reduced in vitro by treatment of the collagen with a reducing agent (e.g., sodium borohydride).

The purity of the collagen fibril matrix according to the teachings of the present disclosure is determined by SDS-PAGE, peptide mapping, amino terminal sequence directly on the collagen polymer or after specific enzyme (bacterial collagenase) or chemical (bromocyan) cleavage. Determination, and can be assessed by any means known in the art including, but not limited to, non-collagen impurity assays. Methods for characterizing the collagen fibril matrix include cation exchange HPLC, natural fluorescence, LC / MS, MS, dynamic light scattering, molecular sieve chromatography, viscosity measurement, circular dichroism, differential scanning calorimetry, trypsin Including, but not limited to, sensitivity, impurity profiling, TEM, SEM, cryo-SEM, confocal microscopy, multiphoton microscopy and atomic force microscopy.

The ratio of probiotics to collagen in step (i) of the method according to the invention is not particularly limiting as long as the number is sufficient so that after step (iii) of the methods of the invention, sufficient number of probiotic microorganisms are found in the biocomposite so as to achieve the desired effect. In one embodiment, the population of probiotic bacteria is contacted with the collagen monomers at a ratio of about 10⁴-10¹⁴ CFU of probiotic microorganisms for each mg of collagen. In one embodiment, the population of probiotic bacteria is contacted with the collagen monomers at a ratio of about 10⁵-10¹³ CFU of probiotic microorganisms for each mg of collagen. In one embodiment, the population of probiotic bacteria is contacted with the collagen monomers at a ratio of about 10⁶-10¹² CFU of probiotic microorganisms for each mg of collagen. In one embodiment, the population of probiotic bacteria is contacted with the collagen monomers at a ratio of about 10⁷-10¹¹ CFU of probiotic microorganisms for each mg of collagen. In one embodiment, the population of probiotic bacteria is contacted with the collagen monomers at a ratio of about 10⁸-10¹⁰ CFU of probiotic microorganisms for each mg of collagen. In one embodiment, the population of probiotic bacteria is contacted with the collagen monomers at a ratio of about 10⁹-10¹⁰ CFU of probiotic microorganisms for each mg of collagen. In one embodiment, the population of probiotic bacteria is contacted with the collagen monomers at a ratio of about 6 × 10⁹ CFU of probiotic microorganisms for each 1.0 mg of collagen.

In step ii) the mixture obtained in step i) is incubated under conditions adequate for the self-assembly of the collagen monomers into collagen fibers, thereby obtaining collagen fibers containing entrapped probiotic bacteria.

The polymerization kinetics can be modulated by controlling parameters such as the pH, the temperature and monomer concentration. In different embodiments, the monomer concentration is as defined above. In preferred embodiments, the parameters of solution flow rate, solution viscosity and substrate rotational velocity are controlled. Typically, the solution which is formed in step (ii) has a pH of about 6 to 9, of about 6.5 to 8.5, of about 7 to 8, of about 7.2 to 7.8, of about 7.4 to 7.7 or of about 7.6.

Typically, the parameters of solution flow rate, solution viscosity and substrate rotational velocity are controlled to produce a shear rate between 1 s⁻¹ and 500,000 s⁻¹, preferably between 10 s⁻¹ to 10,000 s⁻¹. In some embodiments, the solution flow is controlled at a constant rate of about 0.05 to about 1,000 ml/min, preferably between about 0.1 to about 100 ml/min. In other embodiments, the solution viscosity is controlled in the range of about 1 mPa-sec. In one embodiment, the viscosity is about 10 mPa-sec.

Step (ii) of the method according to the invention is allowed to proceed for a predefined time or under continuous monitoring until sufficient percentage of the collagen monomers have assembled into fibers. The structures formed in step (ii) are characterized using well known methods to characterize the microstructural features of the collagen fibril matrix which include environmental or cryostage scanning electron microscopy, transmission electron microscopy, infrared spectroscopy (FTIR), and at the molecular scale by X-ray diffraction.

In one embodiment, step (ii) is allowed to proceed until the structures are formed which, when analysed by FTIR, show peaks at 1030 and 1078 cm⁻¹ corresponding to the C-O stretch of the carbohydrates groups (v(C-O) and broad absorptions bands centered at 1650, 1550 and 1240 cm⁻¹ which arise from amides I, II and III, respectively.

In one embodiment, step (ii) is allowed to proceed until the structures are formed which when analyzed by thermogravimetric analysis (TGA) shows the loss of water between room temperature and 150 °C and the decomposition of collagen between 230 and 600 °C. Alternatively or additionally, the structures formed after step (ii) may be characterized by weight loss between 180 and 240. This applies to Col-Lf bicomposites, which arise from the decomposition of Lf.

In one embodiment, step (ii) is allowed to proceed until the structures are formed which when analyzed by x-ray diffraction analysis show a diffraction pattern characterized by the presence of a broad peak centered at ca. q = 13.25 nm⁻¹ (resulting in a d-spacing of 0.47 nm) associated to the diffuse scattering of the collagen fibers and a Bragg peak at q = 5.46 nm⁻¹ (resulting in a d-spacing of 1.15 nm), which corresponds to the equatorial molecule-molecule averaged separation (i.e., the intermolecular lateral spacing).

Tensile, compressive and viscoelastic properties can be determined by rheometry or tensile testing. All of these methods are known in the art. Collagen features and methods for characterizing collagen features are discussed in ASTM International F3089-14, 2014 West Conshohocken PA. In certain aspects of the present disclosure, the synthetic collagen fibril matrix exhibits a stiffness of at least 5 Pa. In another embodiment, the synthetic collagen fibril matrix exhibits firmness between 5 Pa and 100 GPa. Stiffness can also be referred to as elasticity or linear modulus.

In one embodiment, fibers are formed which show a diameter of between 10 and 900 nm, more preferably between 50 and 800 nm, more preferably between 100 nm and 700 nm, more preferably between 150 and 600 nm and even more preferably between 200 and 500 nm.

In one embodiment, collagen fibrils are formed which show a 67 nm periodic staggered D-banding pattern.

In one embodiment, step ii) of the method according to the invention is performed under continuous stirring. Mechanical stirring is usually employed.

In step (iii), the collagen fibrils containing entrapped probiotic bacteria obtained in step (ii) are maintaining under conditions adequate for the formation of exopolysaccharide (EPS) by the probiotics until the content of EPS in the biomaterial with respect to the collagen content of the biomaterial measured as the sugar content is of at least 2% (w/w).

Typically, "conditions adequate for the formation of exopolysaccharide (EPS)" are conditions which are suitable for the proliferation of the probiotic bacteria which are entrapped in the collagen fibrils. This usually requires maintaining the collagen fibers in a culture medium which is adequate for the growth of the probiotic bacteria which are entrapped in the collagen fibers. Suitable culture medium to be used in step (iii) include any culture medium which is capable of producing cell concentrations around 10⁹ cfu/mL. In preferred embodiments, when the population of probiotic bacteria contains lactic acid bacteria, the culture medium is Man, Rogosa, and Sharpe (MRS) medium, Reinforced Clostridial Medium (RCM), M17 or Brain Heart Infusion (BHI), HANK'S medium, APT medium, LM17 medium, GM17 medium and Elliker medium. In other embodiments, when the population of probiotic bacteria contains Bifidobacterium, the culture media to be used in step (iii) includes MRS, Tryptone Phytone Yeast (TPY), glucose blood liver (BL), Columbia (CLB), Liver cysteine lactose (LCL), modified MRS (mMRS), modified MRS and blood (mMRS + blood), modified BL with blood (mBL), modified RCM (mRCM), RCPB and the like. A description of MRS medium and other media appropriate for the culture of lactic acid bacteria and bifidobacteria can be found in Handbook of Culture Media for Food Microbiology, Vol. 34, edited by Janet E.L. Corry, G.D.W. Curtis, Rosamund M. Baird.

Step (iii) is carried out for as long is needed until the sugars content of in the biocomposite is of at least 2% (w/w) of with respect to the collagen content. The sugar content, which reflects the contents of EPS in the biocomposite is determined using any method known in the art to measure total sugar content in a sample. In a preferred embodiment, the sugar content in the biocomposite is determined using the standard phenol-sulfuric acid method (DuBois, K. A. et al., Anal. Chem. 1956, 28, 350). In one embodiment, step (iii) is allowed to proceed until the sugar content in the biocomposite if of at least 2,5% (w/w), at least 3%, at least 3,5%, at least 4%, at least 4,5%, at least 5%, at least 5,5%, at least 6%, at least 6,5%, at least 7%, at least 7,5%, at least 8%, at least 8,5%, at least 9%, at least 9,5%, at least 10%, at least 11%, at least 12%, at least 13%, at least 14%, at least 15%, at least 16%, at least 17%, at least 18%, at least 19%, at least 20%, at least 25%, at least 30%, at least 35%, at least 40%, at least 50% or more, all these values referred to in w/w with respect to the collagen content in the biocomposite. It will be understood that the progression of step (iii) can also be monitored by SEM by detecting the formation of a network in the biomaterial that corresponds to the EPS.

It will be understood that the method according to the present invention results in a biomaterial in which a substantial fraction of the probiotics entrapped in the collagen scaffold remain viable. Reference herein to a probiotic "remaining viable" after step (iii) of the method according to the invention means that it is possible to culture the probiotics. Although this does not mean that every probiotic cell in the biomaterial obtained after step (iii) remains viable, it is to be construed as that detectable numbers of the probiotic can be cultured.

In different embodiments, after step (iii) at least 100 %, at least 99,9%, at least 99,8%, at least 99,7%, at least 99,6%, at least 99,5%, at least 99,4%, at least 99,3%, at least 99,2%, at least 99,1%, at least 90%, at least 85%, at least 80%, at least 75%, at least 70%, at least 65%, at least 60% or at least 50% of the probiotics in the biomaterial are viable for at least 6 months at 25°C and 60 percent relative humidity, such as at least 24 months, such as at least 20 months, such as at least 16 months, such as at least 12 months, such as at least 8 months, such as at least 5 months, such as at least 4 months, such as at least 3 months, such as at least 2 months, such as at least 1 month, such as at least 14 days or, such as for 14 days to 24 months, such as for 14 days to 16 months, such as for 14 days to 8 months, such as 14 days to 4 months, such as 14 days to 2 months, such as 14 days to 1 month.

Any method known in the art for determining viability of a probiotic population can be used in to determine the content of viable probiotics in the biomaterial, such as the method disclosed in example 2 based on the determination of the reductive capacity of bacteria and the correlation between the activity and the metabolic activity, using an electrochromic polyoxometalate (POM) (González, N. et al. Chem. Commun. 2015, 51). Once reduced, POM exhibits an absorption band in the UV-vis spectrum centered at 820 nm.

### Biocomposites of the invention

The authors of the present invention have found that the method as defined above results in a biocomposite material that shows certain unexpected properties in terms of preserving the viability of the probiotics entrapped therein even after storage under harsh conditions.

Accordingly, in another aspect, the invention relates to a biomaterial obtainable by a method of the invention.

In another aspect, the invention relates to a biomaterial comprising a collagen scaffold, probiotics and exopolysaccharide (EPS). In one embodiment, the collagen scaffold comprises fibrils between 100 and 500 nm diameter. In another embodiment, the collagen scaffold comprises fibrils characterized by a 67 nm periodic staggered D-banding pattern. In another embodiment, the sugar content in the biomaterial with respect to the collagen content of the biomaterial measured is of at least 2% (w/w).

The term "probiotic" has been explained in detail in the context of the method for producing the biomaterial according to the invention and is used with the same meaning in the context of the biomaterial according to the invention. In preferred embodiments, the probiotic is a lactic acid bacteria, a *Bifidobacterium* or a combination of both. In a more preferred embodiment, the Lactobacillus is selected from *L. fermentum*, *L. acidophilus L. crispatus, L. jensenii* and, *L. gasseri* or wherein the Bifidobacterium is selected from *B*. *breve, B. longum, B. bifidum, and B. dentium* or any combination thereof. In a still more preferred embodiment, the probiotic is *L. fermentum* and/or *L acidophilus* and more particularly, *L. fermentum* CECT5716 and/or *L. acidophilus* CECT903 or a combination thereof.

In the biomaterial, a substantial fraction of the probiotics entrapped in the collagen scaffold remain viable In different embodiments, at least 100 %, at least 99,9%, at least 99,8%, at least 99,7%, at least 99,6%, at least 99,5%, at least 99,4%, at least 99,3%, at least 99,2%, at least 99,1%, at least 90%, at least 85%, at least 80%, at least 75%, at least 70%, at least 65%, at least 60% or at least 50% of the probiotics in the biomaterial are viable for at least 6 months at 25 °C and 60 percent relative humidity, such as at least 24 months, such as at least 20 months, such as at least 16 months, such as at least 12 months, such as at least 8 months, such as at least 5 months, such as at least 4 months, such as at least 3 months, such as at least 2 months, such as at least 1 month, such as at least 14 days or, such as for 14 days to 24 months, such as for 14 days to 16 months, such as for 14 days to 8 months, such as 14 days to 4 months, such as 14 days to 2 months, such as 14 days to 1 month.

The term "collagen scaffold", as used herein, refers to a matrix which results from the spontaneous assembly of collagen monomers when placed in a slightly acidic, neutral or slightly alkaline medium. The term "collagen monomer" has been explained in detail above in the context of the method for obtaining the biocomposites and is equally applied to the present case. In a preferred embodiment the collagen scaffold is obtained from type I collagen.

Suitable concentration of probiotics in the biocomposite are of at least 6 ×10¹² CFU per gram of collagen.

In one embodiment, the collagen scaffold is characterized in that it shows peaks at ca. 1030 and 1080 cm⁻¹ corresponding to the C-O stretch of the carbohydrates groups (v(CO)) and broad absorptions bands centered at 1650, 1550 and 1240 cm⁻¹ which arise from amides I, II and III, respectively, when analysed by FTIR. In another embodiment, the collagen scaffold is characterized in that it shows a broad peak centered at ca. q = 13.25 nm⁻¹ and a Bragg peak at q = 5.46 nm⁻¹ resulting in a d-spacing of 0.47 and 1.15 nm, respectively, when analysed by X-Ray diffraction. In another embodiment, the collagen scaffold is characterized in that it shows the loss of water between room temperature and 150 °C and the decomposition of collagen between 230 and 600 °C when analysed by thermogravimetric analysis.

The term "exopolysaccharide" as used herein refers to natural polymers of high molecular weight composed of sugar residues and secreted by microorganisms into their environment. Exopolysaccharides may be referred to interchangeably as extracellular polysaccharides (EPSs). EPSs establish the functional and structural integrity of biofilms, and are considered the fundamental component that determines the physiochemical properties of a biofilm. EPSs constitute 50 percent to 90 percent of a biofilm's total organic matter. Some exopolysaccharides, however, also comprise non-carbohydrate substituents (such as acetate, pyruvate, succinate, and phosphate).

Exemplary exopolysaccharides include, without limitation acetan (*Acetobacter xylinum*), alginate (*Azotobacter vinelandii*), cellulose (*Acetobacter xylinum*), chitosan (Mucorales sp.), curdlan (*Alcaligenes faecalis* var. *myxogenes*), cyclosophorans (*Agrobacterium* sp., *Rhizobium* sp. and *Xanthomonas* sp.), dextran (*Leuconostoc mesenteroides, Leuconostoc dextranicum* and *Lactobacillus hilgardii*), emulsan (*Acinetobacter calcoaceticus*), galactoglucopolysaccharides (*Achromobacter* sp., *Agrobacterium radiobacter, Pseudomonas marginalis, Rhizobium* sp. and Zooglea sp.), gellan (*Aureomonas elodea* and *Sphingomonas paucimobilis*), glucuronan (*Sinorhizobium meliloti*), N-acetyl-glucosamine (*Staphylococcus epidermidis*), N-acetyl-heparosan (*Escherichia coli*), hyaluronic acid (*Streptococcus equi*), indican (*Beijerinckia indica*), kefiran (*Lactobacillus hilgardii*), lentinan (*Lentinus elodes*)*,* levan (*Alcaligenes viscosus, Zymomonas mobilis, Bacillus subtilis*), pullulan (*Aureobasidium pullulans*), scleroglucan (*Sclerotium rolfsii, Sclerotium delfinii* and *Sclerotium glucanicum*), schizophyllan (*Schizophylum commune*), stewartan (*Pantoea stewartii* subsp. *stewartii*), succinoglycan (*Alcaligenes faecalis* var *myxogenes, Sinorhizobium meliloti*), xanthan (*Xanthomonas campestris*), and welan (*Alcaligenes* sp.).

Suitable EPS found in the biocomposites according to the present invention include any EPS that is produced by one or more of the probiotic strains forming the probiotic cell population. In the particular case that the probiotic cell population comprises lactic acid bacteria (LAB), the EPS forming part of the biocomposites can be a homopolysaccharide (HoPS), a heteropolysaccharide (HePS) or a mixture thereof.

The biomaterial according to claim 14, wherein the HoPS is selected from α-glucans, β-glucans and β-fructans, and the HePS comprises any of D-glucose, D-galactose, L-rhamnose and N-acetylated monosaccharides selected from N-acetyl-glucosamine (GluNAc), N-acetyl-galactosamine (GaINAc), fucose, glucuronic acid, glycerol and mannose.

HoPS are composed of one type of constituting monosaccharides (D-glucopyranose or D-fructofuranose) and include, without limitation, from α-glucans, β-glucans and β-fructans, and the HePS comprises any of D-glucose, D-galactose, L-rhamnose and N-acetylated monosaccharides selected from N-acetyl-glucosamine (GluNAc), N-acetyl-galactosamine (GaINAc), fucose, glucuronic acid, glycerol and mannose.

In other embodiments, the EPS forming part of the biocomposites according to the invention include one or more of the HoPS summarized in Table 2 of Ruas-Madiedo et al. (International Dairy Journal, 2002, 12, 163-171) and which include:
- α-D-glucans, including dextran, mutan, alternan,
- β-D-glucans,
- Fructans, including Levans and Inulin-like
- Polygalactan

HePS are composed of a backbone of repeated subunits, that are branched (at positions C2, C3, C4, or C6) or unbranched and that consist of three to eight monosaccharides, derivatives of monosaccharides or substituted monosaccharides.

In some embodiments, the HoPS and HePS forming part of the biocomposites according to the present invention include any of the molecules which are shown in Table 1 in de Vuyst et al. (International Dairy Journal, 2001, 11, 687-707).

These include HoPS and HePS in which the repeating units comprise glucose (Glc), GIcNAc (N-acetylglucosamine), galactose, (Gal), GalNAc (N-acetylgalactosamine), rhamnose (Rha) and fucose (Fuc) in different combinations and in which the repeating units are trisaccharides, tetrasaccharides, pentasaccharides, hexasaccharides, pentasaccharides and octasaccharides.

In other embodiments, the HoPS and HePS forming part of the biocomposites according to the present invention include any of the molecules which are shown in Table 4 in Vaningelgem et al. (Applied and Environmental Microbiology, 2004, 70:900-912). These include EPSs composed of galactose and glucose (Group I), galactose and rahmnose (Group II), galactose, glucose and N-acetylgalactosamine (Group III), galactose, glucose and rahmnose (Group IV), galactose, glucose, N-acetylgalactosamine and rahmnose (Group V) or galactose, glucose and N-acetylglucosamine (Group VI).

In other embodiments, the HePS forming part of the biocomposites according to the present invention include any having any of the repeating units summarized in Table 2 of Ruas-Madiedo et al. (International Dairy Journal, 2002, 12, 163-171).

In one embodiment, the EPS forming part of the biocomposites according to the invention is selected from dextran, levan, mutan, alternan, reuteran and inulin-like. In another embodiment, the EPS is dextran, levan or a combination thereof.

The contents of EPS in the biocomposites according to the invention is not particularly limiting. The content of EPS can be determined by measuring the sugar content in the biocomposite determined using any method known in the art to measure total sugar content in a sample. In some embodiments, the sugar content in the biocomposite may be of at least 2%, at least 2.5%, at least 3%, at least 3.5%, at least 4%, at least 4.5%, at least 5%, at least 5.5%, at least 6%, at least 6.5%, at least 7%, at least 7.5%, at least 8%, at least 8.5%, at least 9%, at least 9.5%, at least 10%, at least 11%, at least 12%, at least 13%, at least 14%, at least 15%, at least 16%, at least 17%, at least 18%, at least 19%, at least 20%, at least 25%, at least 30%, at least 35%, at least 40%, at least 50% or more, all these values referred to as w/w with respect to the collagen content in the biocomposite.

### Medical uses and pharmaceutical compositions of the biocomposites of the invention

The authors of the present invention have found that the biocomposites of the invention can be attached to porcine mucine, said adhesion being partially dependent on the presence of collagen in the solution. Moreover, the biocomposites of the invention are also capable the of restoring the healthy conditions from a fluid which mimics the fluid that appears in bacterial vaginosis by inducing a change of the pH of 5 which appears in pathological conditions to a value of pH of 4 which corresponds to healthy conditions. Without wishing to be bound by any theory, this pH shift is certainly due to excreted lactic acid by the probiotic bacteria, in agreement with the previous evidences that the bacteria remain alive and active once entrapped into the collagen matrix. Interestingly, the drop in pH with *Lf* and *La* without collagen was much slower and did not reach the healthy pH value of 4.0.

Accordingly, in another aspect, the invention relates to a biomaterial according to the invention for use in medicine. In another embodiment, the invention relates to a biomaterial or biocomposite according to the invention for use in the treatment of a vaginal dysbiosis, preferably a vaginal infection. In another aspect, the invention relates to a method for the treatment or prevention of a vaginal dysbiosis, more preferably a vaginal infection, in a subject which comprises the administration of the biomaterial or biocomposite material according to the present invention.

As used herein "preventing" or "prevention" refers to any methodology where the disease state does not occur due to the actions of the methodology (such as, for example, administration of a probiotic and/or a prebiotic as described herein). In one aspect, it is understood that prevention can also mean that the disease is not established to the extent that occurs in untreated controls. For example, there can be a 5, 10, 15, 20, 25, 30, 35, 40, 50, 60, 70, 80, 90, or 100% reduction in the establishment of disease frequency relative to untreated controls. Accordingly, prevention of a disease encompasses a reduction in the likelihood that a subject will develop the disease, relative to an untreated subject (e.g. a subject who does not receive a probiotic and/or a prebiotic as described herein).

"Treatment," "treat," or "treating" means a method of reducing the effects of a disease or condition. Treatment can also refer to a method of reducing the disease or condition itself rather than just the symptoms. The treatment can be any reduction from pre-treatment levels and can be but is not limited to the complete ablation of the disease, condition, or the symptoms of the disease or condition. Therefore, in the disclosed methods, treatment" can refer to a 10%, 20%, 30%, 40%, 50%, 60%, 70%, 80%, 90%, or 100% reduction in the severity of an established disease or the disease progression. For example, a disclosed method for reducing the effects of a vaginal infection is considered to be a treatment if there is a 10% reduction in one or more symptoms of the disease in a subject with the infection when compared to pre-treatment levels in the same subject or control subjects. Thus, the reduction can be a 10, 20, 30, 40, 50, 60, 70, 80, 90, 100%, or any amount of reduction in between as compared to native or control levels. It is understood and herein contemplated that "treatment" does not necessarily refer to a cure of the disease or condition, but an improvement in the outlook of a disease or condition (e.g., bacterial vaginosis).

The term "dysbiosis" or "dysbiotic condition" is defined as a state in which the microbiota produces harmful effects via (a) qualitative and quantitative changes in the content or amount of the microbiota itself, (b) changes in their metabolic activities; and/or (c) changes in their local distribution. Specifically, dysbiosis of the vagina is defined as an aberration of the healthy state. A healthy state is defined in this case as a condition with a relatively low susceptibility to sexually transmitted diseases. It has been widely accepted that the activity of Lactobacillus spp. contributes to maintain this low susceptibility through the protection of the vaginal environment against pathogens by the production of lactic acid, resulting in a low pH. Hence, vaginal dysbiosis is, amongst others, characterized by the presence of a relative high pH.

In one embodiment, the vaginal dysbiosis is a vaginal infection.

The term "vaginal infection", as used herein, refers to a disorder or disease which results from the undesired growth of a pathogen in the vagina selected from the group comprising: *Candida glabrata*, *Candida parapsilosis*, *Candida krusei*, *Candida tropicalis*, *Gardnerella vaginalis, Trichomonas vaginalis, Neisseria gonorrhoeae, Escherichia coli,* Herpes simplex and *Hemophilus ducreyie.* The term includes, without limitation, candidiasis, vaginitis, vulvovaginitis and bacterial vaginosis.

In one embodiment, the biomaterial for use according to the present invention is used for the treatment of an infection selected from bacterial vaginosis, trichomonas vaginitis or candidiasis. In yet another embodiment, the biomaterial for use according to the present invention is used in the treatment of an infection caused by a bacteria, yeast or protozoan parasite.

In one embodiment, the infection is caused by overgrowth of yeast in the vulvovaginal area, in which case the compositions according to the present invention are used for "vaginal yeast infection". Vaginal yeast infections are usually caused by excessive growth of Candida yeast, such as, but not limited to, commensal organism *Candida albicans.* Other Candida species implicated in vaginal yeast infections include, but are not limited to, *Candida glabrata*, *Candida parapsilosis*, *Candida krusei* and *Candida tropicalis.* Vaginal yeast infection can be referred to as "vaginal candidiasis" or "candidal vaginitis." Vaginal yeast infections can also be caused by non-Candida yeast, such as Trichosporon species, such as Trichosporon beigelii, and Sacharomyces cereviasae. The symptoms of vaginal yeast infections are vaginal irritation, discharge and intense itchiness of the vagina and the vulva. As is the case with BV, broad-spectrum antibiotics, spermicides, hormones, and other factors, not yet fully understood, may cause disruption of normal bacterial flora, leading to vaginal yeast infections. Vaginal yeast infections are also common in immunocompromised subjects

In another embodiment, the infection is bacterial vaginosis.

The term "bacterial vaginosis," abbreviated as "BV," and similar terms are to be understood in the broad sense as the alterations of vaginal bacterial flora composition in a female subject, as compared to a baseline, reference or "normal" vaginal bacterial flora composition. BV is not limited to a specific vaginal bacterial flora composition or any particular symptoms observed in a particular female subject or population of female subjects. The term, "vaginosis" defined herein comprise a vaginosis selected from bacterial vaginosis, fungal vaginitis or Tricomonas vaginitis, preferably, a vaginosis caused Gardnerella vaginalis, *Bacterioid fragilis*, *Tricomonas vaginalis*, *Candida albicans, Streptococcus agalactiae, Streptococcus aureus, Staphylococcus aureus, Neisseria gonorrhoeae, Escherichia coli, Enterobacter cloacae, Pseudomonas aeruginosa*, or *Salmonella typhimurium*, and the like. In one embodiment, the bacterial vaginosis is caused by overgrowth of normal vagina flora selected from *Gardnerella vaginalis* or Mobiluncus spp.

Processes for using the probiotic vaginal formulations are provided herein. For example, in some embodiments, the probiotic vaginal formulations are used to acidify or maintain the acidity of the vaginal environment. Accordingly, the probiotic vaginal formulations may be used in the methods of modulating acidification, including, increasing the acidity, maintaining the acidity, or lowering vaginal acidity or pH. In some embodiments, the probiotic vaginal formulations are used to establish or improve vaginal flora, for example, by increasing the levels of Lactobacilli in the vaginal flora or decreasing the levels of yeast, such as Candida yeast. The probiotic vaginal formulations may also be used in the methods of treating, alleviating, preventing or reducing the likelihood of urogenital infections or inflammatory conditions, including vaginitis, urinary tract infections, vaginal atrophy and post-surgical inflammation in a subject. For example, the probiotic vaginal formulations may be used in the methods of treating, alleviating, preventing or reducing the likelihood of vaginitis or its symptoms, such as vaginal dryness, burning, or itching in a subject. In the context of the above methods, vaginitis may be various etiologies, such as, but not limited to, BV, yeast infection or vaginal atrophy. Vaginitis or vaginitis-related or vaginitis-like symptoms may also be of unknown etiology. In another example, the probiotic vaginal formulations may be used in the methods of treating, alleviating, preventing or reducing the likelihood of vaginal dryness of various etiologies, such as, but not limited to, BV, yeast infection, trauma, medication side-effect or vaginal atrophy. In another example, the probiotic vaginal formulations may be used in the methods of treating, alleviating, preventing or reducing the likelihood of urogenital inflammation, including vulvovaginal inflammation of various etiologies, such as, but not limited to, infection, irritation, trauma, such as post-surgical or childbirth trauma. One or more uses of the probiotic vaginal formulations may be described as suitable for improving, maintaining or achieving vaginal health.

Processes or methods of using the probiotic vaginal formulations include the step of vaginally administering to the subject a probiotic vaginal formulation in an effective amount. The effective amount may vary based on the desired effect, the specific formulation, the dosage form, the dosing regimen, and other factors. For example, the formulation, in capsule unit dosage form, may be administered in an amount of 1-5 capsules per day, including, for example, 1 or 2 capsules per day. The period of administration may be for at least 1 day, at least 3 days, at least 6 days, or as prescribed by a physician or until the improvement or reduction of the symptoms is achieved

The therapeutic methods according to the present invention comprise the administration of a therapeutically effective amount of the biomaterial or probiotic biocomposites of the invention to a subject in need thereof.

The term "therapeutically effective amount", as used herein, refers to the sufficient amount of the bacterium or culture of the invention to provide the desired effect and will generally be determined by, among other causes, the characteristics of the bacterium or culture of the invention itself and the therapeutic effect to be achieved. It will also depend on the subject to be treated, the severity of the disease suffered by said subject, the chosen dosage form, etc. For this reason, the doses mentioned in this invention must be considered only as guides for the person skilled in the art, who must adjust the doses depending on the aforementioned variables. In an embodiment, the effective amount produces the amelioration of one or more symptoms of the disease that is being treated, for example, the normalization of the levels of the metal that was deficient in the subject.

The term "subject" is used herein to describe a human or an animal. In the context of the embodiments of the present products, formulations, and processes, "subject" denotes a female mammal, such as a human, to whom the compositions are vaginally administered. Female subjects can have or be at risk of developing a particular disease or condition, for example, a urogenital infection, such as vaginitis. For example, female subjects may be at risk of recurrence of a urogenital infection or vaginitis, based on their medical history, antibiotic use, spermicide use or lifestyle (for example, sexual habits). Female subjects may also have or wish to prevent a urogenital infection or vaginitis or symptoms thereof, a suspected urogenital infection or suspected vaginitis, and urogenital infection-like or vaginitis-like symptoms, even in the absence of a formal diagnosis or full-blown urogenital infection or vaginitis.

In order for the biomaterial or biocomposite material of the invention to be administered, it has to be formulated as product suitable for vaginal administration, i.e. as a vaginal product or vaginal formulation. Thus, in another embodiment, the invention relates to a pharmaceutical composition comprising the biomaterial according to the invention and a pharmaceutical acceptable excipient suitable for vaginal administration.

The term "vaginal product" or "vaginal formulation" is used herein to refer to compositions of matter in a dosage form suitable for vaginal administration. The terms "probiotic vaginal product" and "probiotic vaginal formulation" refer to a composition of matter in a dosage form suitable for vaginal administration and comprising a probiotic. Some examples of vaginal formulations are suppositories (including, but not limited to, capsules and tablets), tampons, solutions, powders, ointments, creams and aerosol foams.

The term "vaginal administration" includes "transvaginal administration" and "intravaginal administration," which refer to routes of administration, in which a substance, a composition or a formulation is applied inside a vaginal cavity or vulvovaginal area or through the vagina to other parts of the urogenital tract.

Probiotic compositions for topical administration may be applied in the form of cremes or ointments. As such they will ideally comprise an oily substance originating from vegetable, marine or animal sources. Suitable liquid oil includes saturated, unsaturated or polyunsaturated oils. By way of example, the unsaturated oil may be olive oil, corn oil, soybean oil, canola oil, cottonseed oil, coconut oil, sesame oil, sunflower oil, borage seed oil, syzigium aromaticum oil, hempseed oil, herring oil, cod-liver oil, salmon oil, flaxseed oil, wheat germ oil, evening primrose oils or mixtures thereof, in any proportion. These creams or ointments may further comprise poly-unsaturated fatty acids. In one or more embodiments, said unsaturated fatty acids are selected from the group of omega-3 and omega-6 fatty acids. Examples of such polyunsaturated fatty acids are linoleic and linolenic acid, gamma-linoleic acid (GLA), eicosapentaenoic acid (EPA) and docosahexaenoic acid (DHA). Such unsaturated fatty acids are known for their skin-conditioning effect, which contribute to the therapeutic benefit of the composition. Thus, the composition can include at least 6 percent of an oil selected from omega-3 oil, omega-6 oil, and mixtures thereof. Also usable are the essential oils, which are also considered therapeutically active oils, which contain active biologically occurring molecules and, upon topical application, exert a therapeutic effect, which is conceivably synergistic to the beneficial effect of the probiotic mixture in the composition. Another class of therapeutically active oils includes liquid hydrophobic plant-derived oils, which are known to possess therapeutic benefits when applied topically. Silicone oils also may be used and are desirable due to their known skin protective and occlusive properties. Suitable silicone oils include non-volatile silicones, such as polyalkyl siloxanes, polyaryl siloxanes, polyalkylaryl siloxanes and polyether siloxane copolymers, polydimethylsiloxanes (dimethicones) and poly(dimethylsiloxane)-(diphenyl-siloxane) copolymers. These are chosen from cyclic or linear polydimethylsiloxanes containing from about 3 to about 9, preferably from about 4 to about 5, silicon atoms. Volatile silicones such as cyclomethicones can also be used. Silicone oils are also considered therapeutically active oils, due to their barrier retaining and protective properties.

For vaginal application the probiotic composition may be in the form of a vaginal capsule or vaginal tablet. The term "capsule" refers to a hard shell pharmaceutical capsule. The capsule consists of a body and cap and may comprise a fill formulation containing the probiotic composition. Capsules suitable for use according to the invention include, without limitation NPcapsCR' available from Capsugel which contain pullulan, carageenan and potassium chloride, as well as capsules described in US Patent No. 8, 105,625 and US Patent Application Publication No. 2005/0249676. In one aspect, capsules for use according to the invention comprise pullulan with a molecular weight between about 50 to 500 kDa, between 100 to 400 kDa, between about 150 to 300 kDa and preferably between about 180 and 250 kDa. In another aspect, capsules for use according to the invention comprise pullulan from about 50 percent to about 100 percent by weight (unfilled capsule). In other aspects, the capsules comprise about 60 to 90 or 70 to 90, or 80 to 90 wt percent pullulan. Preferably the capsules comprise about 85 to 90 wt percent pullulan. Capsules for use according to the invention may further comprise (in addition to pullulan) one or more gelling agents (e.g. hydrocolloids or polysaccharides such as alginates, agar gum, guar gum, carob, carrageenan, tara gum, gum arabic, pectin, xanthan and the like); salts comprising cations such as K, Li, Na, NH4, Ca, Mg; and/or surfactants such as sodium lauryl sulphate, dioctyl sodium sulfosuccinate, benzalkonium chloride, benzethonium chloride, cetrimide, fatty acid sugar esters, glycerl monooleate, polyoxyethylene sorbitan fatty acid esters, polyvinylalcohol, dimethylpolysiloxan, sorbitan esters or lecithin.

Capsules for use according to the invention may further comprise one or more plasticizing agents (e.g. glycerol, propylene glycol, polyvinyl alcohol, sorbitol, maltitol and the like); dissolution enhancing agents (e.g. maltose, lactose, sorbitol, mannitol, xylitol, maltitol and the like); strengthening agents (e.g. polydextrose, cellulose, maltodextrin, gelatin, gums and the like); colorants, and/or opacifiers as described in US Patent No. 8, 105,625. In a preferred embodiment, the capsule comprises pullulan in an amount of 85 percent to 90 percent by weight, potassium chloride in an amount of 1.0 percent to 1.5 percent by weight, carrageenan in an amount of 0.1 percent to 0.4 percent by weight, one or more surfactants in an amount of 0.1 percent to 0.2 percent by weight and water in an amount of 10 percent to 15 percent by weight.

The dosage forms of the probiotic vaginal probiotic formulations are suitable for vaginal administration and are selected to deliver an effective amount of the vaginal probiotic formulation. In some embodiments, the probiotic vaginal formulation is supplied as a powder and can be administered by a suitable applicator. In this case, the probiotic vaginal formulation may be supplied as a component of a kit, which includes an applicator. The probiotic vaginal formulation may be pre-packaged in the applicator, or supplied as a separate item of the kit. In some other embodiments, the probiotic vaginal application are incorporated into vaginal tampons. A kit comprising the tampons optionally includes one or more applicators. Suitable dosage forms also include vaginal suppositories, including capsules and tablets, which can be administered by with or without a suitable applicator. The choice of the dosage form depends on a variety of factors. For example, the chosen dosage form should ensure stability of the formulation's ingredients during storage, convenient administration and quick delivery of the formulation in the vaginal environment. In particular, the dosage form of the probiotic vaginal formulations should not detrimentally affect viability or allow premature (prior to administration) reconstitution of the probiotic components of the formulation. To this end, the dosage form should not contain water. At the same time, the dosage form should allow for quick dispersion or dissolution of the formulation's ingredients in the vaginal environment upon administration. For example, if a tablet or a capsule is chosen as a dosage form, it should be formulated to disintegrate quickly when administered into vagina.

Additionally, the administration can be chronic or intermittent, as deemed appropriate by the supervising practitioner, particularly in view of any change in the disease state or any undesirable side effects. "Chronic" administration refers to administration of the composition in a continuous manner while "intermittent" administration refers to treatment that is done with interruption. The effective amount of colony forming units (CFU) for the strains in the composition will be determined by the skilled in the art and will depend upon the final formulation. For instance, when administered orally without any other active agent, the total amount of the strains of the invention is present in the composition in a single dose in amount giving an effective daily dose of from 10⁷ to 10¹² CFU, according to the current legislation, preferably from 10⁹ to 10¹¹ CFU and, when administered vaginally or rectally, in an amount giving an effective daily dose of from 10³ to 10¹² CFU, preferably from 10⁵ to 10¹⁰ CFU. The term "colony forming unit" ("CFU") is defined as number of bacterial cells as revealed by microbiological counts on agar plates. Food supplements usually contain probiotic strains in an amount ranging from 10⁷ and 10¹² CFU/g. In a particular embodiment, the composition of the invention is a food supplement for daily doses comprising between 10⁹-10¹¹ CFU/g. The term "pharmaceutical product" is understood in its widely meaning in this description, including any composition that comprises an active ingredient, in this case, the strains of the invention preferably in form of composition, together with pharmaceutically acceptable excipients. The term "pharmaceutical product" is not limited to medicaments. The term "pharmaceutically acceptable" as used herein pertains to compounds, materials, compositions, and/or dosage forms which are, within the scope of sound medical judgment, suitable for use in contact with the tissues of a subject (e.g. human) without excessive toxicity, irritation, allergic response, or other problem or complication, commensurate with a reasonable benefit/risk ratio. Each carrier, excipient, etc. must also be "acceptable" in the sense of being compatible with the other ingredients of the formulation. Suitable carriers, excipients, etc. can be found in standard pharmaceutical texts.

The invention is defined by way of the following aspects:
The invention is explained below by way of the following examples which are to be construed as merely illustrative and not limitative of the scope of the invention.

### EXAMPLES

### EXPERIMENTAL SECTION

*Reagents, bacteria sources and collagen:* High purity reagents were purchased from Sigma-Aldrich. All solutions were prepared with ultrapure deionized water (18 MΩ·cm). *Lactobacillus fermentum* (*Lf*, CECT5716) powder was obtained from Biosearch Life S.A. *Lactobacillus acidophilus* (*La*, CECT903, Moro 1900) was purchased from CECT (Colección Española de Cultivos Tipo). De Man-Rogosa-Sharpe (MRS) medium was purchased from Thermo Scientific. Type I collagen was extracted from equine tendon using the standardized manufacturing method of OPOCRIN S.p.A. (Corlo di Formigine, Modena, Italy) as described by Tampieri, A. et al. (J. Biomed. Mater. Res., 2003, 67A, 618).

*Entrapment of Lactobacilli bacteria in the collagen matrix: La and Lf* were separately inoculated in 100 mL of MRS broth and incubated for 24 h at 37 °C under continuous stirring (180 rpm). Subsequently, bacteria were collected by centrifugation (10000 g, 5 minutes), washed with saline solution (0.9% NaCl in water) and resuspended in phosphate buffered saline (PBS, pH 7.4) to obtain a bacteria suspension with 2·10⁹ Colony Forming Unit (CFU) mL⁻¹. Then, 3 mL of bacteria suspension was mixed with 1 mL of collagen solution (1.5 mg mL⁻¹, pH 3.0) triggering to the formation of a 3D matrix of self-assembled collagen fibers in the presence of the probiotic. The collagen matrices with the entrapped bacteria were maintained for 30 minutes at 37 °C under continuous stirring (180 rpm) and then, washed with 10 mL of saline solution (0.9% NaCl in water) to remove the non-entrapped bacteria.

*Preparation of col-Lf and col-La biocomposites:* The collagen matrices with the entrapped bacteria were then incubated in 10 mL of MRS broth for 6 h at 37 °C. The resulting materials, referred to as col-Lf and col-La, were washed with saline solution and stored until further ex-situ characterizations.

*Ex-situ characterization of col-Lf and col-La biocomposites by Field Emission Scanning Electron Microscopy (FESEM):* Collagen matrices (with and without probiotics) and Col-La and Col-Lf biocomposites were fixed in 1 mL of cacodylate buffer (0.1 M, pH 7.4) containing 2.5% of glutaraldehyde at 4 °C. After 24 hours, the samples were washed 3 times (30 min at 4 °C) with cacodylate buffer. The sample was stained with osmium tetroxide solution (1 % v/v) for 2 hours in the dark and then repeatedly rinsed with Milli-Q water to remove the excess of osmium. Samples were then dehydrated at room temperature with ethanol/water mixtures of 50%, 70%, 90% and 100% (v/v) for 20 min each, being the last concentration repeated three times and dried at the CO₂ critical point. Finally, dehydrated samples were mounted on metal stubs with conductive adhesive, covered with a thin carbon film and analyzed using a FESEM (Zeiss SUPRA40V, Center for Scientific Instrumentation, University of Granada).

*Ex-situ characterization of col-Lf and col-La biocomposites by Fourier-Transform Infrared (FTIR) spectroscopy:* FTIR spectra were collected on a Bruker Tensor 27 spectrometer under Attenuated total reflection (ATR) configuration with a resolution of 3 cm⁻¹ by accumulation of 100 scans covering the 4000 - 400 cm⁻¹ spectral range.

*Ex-situ characterization of col-Lf and col-La biocomposites by Thermogravimetry analyses (TGA):* TGA were performed using a Mettler-Toledo TGA/DSC1 thermal balance (Mettler-Toledo, Center for Scientific Instrumentation, University of Granada) with a heating rate of 5 °C/min up to 900 °C in nitrogen flow.

*Ex-situ characterization of col-Lf and col-La biocomposites by Synchrotron X-Ray Diffraction:* Data were collected on a 1.0 mm diameter glass capillary filled with either dry collagen or collagen/bacteria matrices at the X04SA-MS Beamline of the Swiss Light Source (SLS) of the Paul Scherrer Institut (Villigen, Switzerland). The beam energy was set at 16 keV (operational wavelength, A = 0.77627 Å). Data were collected with the aid of the position sensitive single-photon counting MYTHEN II detector. The samples were freeze-dried at -40 °C (LyoQuest, Telstar) overnight prior FTIR, TGA and X-Ray diffraction characterizations.

*EPS quantification:* The standard phenol-sulfuric method was used for EPS quantification (DuBois et al., Anal. Chem. 1956, 28, 350). Samples were immersed in 1 mL of saline solution containing with 500 µL of phenol (5% in water), and 2.5 mL of sulfuric acid and maintained in a water bath at 30 °C for 20 min. Absorbance of each sample was measured at 490 nm. EPS concentration was determined using the absorption coefficients obtained from a calibration curve and expressed in mg of sugar per g of collagen. Calibration curves were performed using a mixture of dextran and levan, the most characteristics components of the EPS excreted by lactobacilli. Each sample was tested in triplicate.

*In vitro viability* assay: Bacterial viability in Col-Lf was qualitatively evaluated with *Bac*Light^{™} Bacterial Viability kit (Thermo-Fisher) following manufacturer's instructions. This assay consists in combining membrane impermeable DNA-binding stain, i.e. propidium iodide (PI), with membrane- permeable DNA-binding counterstain, SYTO9, staining dead and live and dead bacteria, respectively. Confocal laser scanning microscopy (CSLM) images were collected with a Nikon Eclipse Ti-E A1 microscope equipped with 60x oil immersion objective. Images were analyzed with NIS Elements software. For acquiring SYTO 9 signals, 488 nm laser and 505-550 nm emission filter was used. For PI, 561 nm laser and 575 nm long-pass emission filter was used. The reconstruction of the tridimensional image was obtained with 39 z-sections.

*Bacterial activity of col-Lf and col-La after storing at 4* °C: Collagen-probiotic biocomposite and the corresponding controls (naked probiotics) were stored at 4 °C for 2 weeks, 1 month and 2 months. After this time, samples and controls were separately added to 10 mL of MRS broth media and incubated at 37 °C and 180 rpm for 1 hour. Then, all samples were washed twice (3000 g, 5 min) with saline solution (0.9% NaCl in water). Subsequently, 10 mL of 1/10 diluted MRS broth media were added and incubated at 37 °C and 180 rpm. 1/10 diluted MRS broth was used to avoid auto-reducing ability of non-diluted MRS broth. Aliquots of 1 mL were sequentially collected from each of the cultures' supernatants at scheduled times: 0, 2, 4, 6, 8 and 24 h. Aliquots were centrifuged (3000 g, 5 min) to remove any residual bacteria. The fermentation activity of the encapsulated living materials and the corresponding controls were monitored by pH measurements. To confirm the higher viability of collagen bacteria systems with respect to non-entrapped bacteria, we applied a procedure developed by us consisting on correlating the bacterial proliferation to its reductive capacity against the electrochromic polyoxometalate (POM), [P₂Mo^{VI} O₆2]⁶⁻) (González et al., Chem. Commun. 2015, 51). 190 µL of each aliquot were added to a well containing 10 µL of a 10 mM solution of POM. Samples were left in dark at room temperature. Then, they were irradiated with UV light (365 nm) for 10 minutes and then the absorbance was measured in a Tecan's NanoQuant plate reader. Same experiments were performed on materials stored at room temperature (fresh samples). In addition, controls of free col were used to confirm that this material itself is not able to decrease pH or reduce POM. Each aliquot was tested in triplicate.

*In vitro* assay *of bacterial adhesion*: The adhesion of col-probiotics and probiotics was assayed according to the method previously reported with slight modifications.[25,26] In brief, the wells of Maxisorp plates (Nunc, Roskilde, Denmark) were incubated overnight at 4 °C with 100 µL of a 5 mg mL⁻¹ solution of porcine mucin (Sigma-Aldrich) in PBS pH 7.4. After immobilization, wells were washed three times with PBS and blocked with 2% BSA in PBS for 1 h. Bacterial pellets from 5 mL fractions of bacterial culture were collected by centrifugation (6000 g, 5 minutes), washed three times with PBS and resuspended in 5 mL of PBS. Upon removal of the BSA solution and washing three times the wells with PBS, the bacterial suspension (100 µL, 10⁸ CFU mL⁻¹) was added. In the case of collagen-bacteria matrix samples, 25 µL of collagen (50 mg mL-1 in 0.1 M acetic acid) were added. The plate was incubated with *Lf* or La on an orbital shaker at 180 rpm for 2 h at 37 °C. Non- adhered bacteria were removed by washing the wells three times with 100 µL of PBS, and the samples were fixed at 60 °C for 20 min. Afterwards, the fixed cells were stained with crystal violet (100 µL per well, 0.1% solution) for 45 min at room temperature. Finally, the wells were washed twice with 150 µL of PBS and the stain bound to the bacteria was dissolved in 50 µL of citrate buffer (pH 4.3) for 1 h. The absorbance was measured at 590 nm using the microtiter plate reader (NanoQuant). Results were expressed as the mean of triplicates with a total volume of 200 µL, after collecting four replicates of 50 µL in one well. Controls consisted of mucin and mucin-collagen, and samples were mucin-*Lf*, mucin-*La*, mucin-col-*La* and mucin-col-*Lf*, blocked or not with BSA.

*Bacterial activity of col-Lf and col-La in a simulant BV fluid:* The collagen-probiotic samples and controls were separately added to 10 mL of simulant BV fluid and incubated at 37 °C and 180 rpm. 1 L of BV fluid, prepared according to the protocol previously reported (D. H. Owen, D. F. Katz, Contraception 1999, 59, 91.) with slight modifications, contains NaCl, 3.51 g; KOH, 1.40 g; Ca(OH)₂, 0.222 g; bovine serum albumin, 0.018 g; urea, 0.4 g; glycerol, 0.16 g; glucose, 10 g; lactic acid, 2 g; and acetic acid, 1 g. The pH was of 5.00. Aliquots of 1 mL were sequentially collected from each of the cultures' supernatants at scheduled times: 0, 2, 4, 6, 8 and 24 h. Aliquots were centrifuged (3000 g, 5 min) to remove any residual bacteria. The pH of the entrapped living materials and the corresponding controls was measured to demonstrate the capacity of restoring the pH of the media. Each aliquot was tested in triplicate.

*Statistical analysis*: Results are expressed as average ± standard error of the mean (S.E.M.). Statistical comparisons were performed using one-way ANOVA and Bonferroni's post hoc test with GraphPad Prism software (version 6.0). A p < 0.05 was considered significant values.

### EXAMPLE 1: Collagen matrix as a scaffold for probiotics

Acidic solution containing collagen monomers was mixed with the probiotics (Lf or La) in PBS buffer (pH 7.4). The instantaneous pH increase induces the collagen self-assembly into a high-density matrix of fibers with several hundred microns in length, and diameters between 200 and 500 nm (Figure 1a,b), showing the characteristic D-banding pattern (67 nm). As it is evident from the scanning electron microscopy (SEM) micrographs (Figure 1c-d), using this simple one-pot procedure, *Lf* (Figure 1c,d) and *La* (Figure 2) probiotics were successfully entrapped into the network of collagen fibers. Further, the presence of probiotics did not alter the pH-induced structure and assembly of the collagen fibers, which were similar to those obtained in PBS without bacteria (Figure 1a,b).

Interestingly, when both probiotic-containing collagen materials were incubated in an optimal probiotic medium, such as MRS, the bacteria successfully proliferated and produced biofilms, which decorated the collagen fibers (Figure 1e-f and 2). It is interesting to note that these kinds of bacteria excrete exopolysaccharides (EPS). EPS play an important role in the formation of the biofilm, which is essential for bacteria to create a growth-conducive microenvironment in a substrate, in this case, the collagen fibers. The excellent integration of bacteria into the collagen matrix, witnessed by the formation of biofilms, is evident from Figure 1e-f, showing how bacteria adhere to collagen fibers through their EPS (Figure 1f and 2). These materials (col-*Lf* and col-*La*, hereafter) are in fact hybrid biocomposites formed by three constituents: the collagen matrix, the probiotic and their bacterial EPS, being the latter the natural 'glue' of the other two components. This class of biocomposites has never been reported.

The amount of EPS in the biocomposites col-*Lf* and col-*La* were quantified by a standard sugar test (see Experimental Section). Values of 98 and 93.3 mg per gram of collagen were obtained for col-*Lf* and col-*La*, respectively. Interestingly, the initial materials obtained by incubating probiotics and collagen in PBS contained much lower sugar levels (10 and 18.6 mg per gram of collagen, respectively). The drastic increase of sugar in the final biocomposites col-*Lf* and col-*La* is certainly due to the biofilm formation in the optimum MRS medium, in agreement with SEM images.

The biocomposite was further characterized at the macroscale by infrared spectroscopy (FTIR) and thermogravimetric analysis (TGA), and at the molecular scale by synchrotron X-ray diffraction (Figure 3). FTIR spectra and TGA of col-*Lf* show the main fingerprints of collagen fibers along with some signals assignable to the presence of bacteria (Figure 3a and b). Diffraction patterns of collagen and col-*Lf* were similar (Figure 3c) with a broad peak centered at ca. *q* = 13.25 nm⁻¹ due to the diffuse scattering of the collagen fibers. The Bragg peak at *q* = 5.46 nm⁻¹ corresponds to the equatorial molecule-molecule averaged separation (*i.e.*, the intermolecular lateral spacing) of the collagen triple-helix. This peak results in a *d*-spacing of 1.15 nm, which is in good agreement with the corresponding *d*-spacing of collagen in dry non-mineralized biological tissues and confirms that bacteria do not affect the macromolecular assembly of collagen fibers.

Standard live/dead test based on SYTO9 (green)/propidium iodide (red) demonstrated that *Lf* and La bacteria were alive in the col-*Lf* and col-La materials (Figure 4). As it is evident from Figure 4, the majority of *Lf* probiotics remain alive (green), with only a few dead bacteria found (red). Furthermore, side-view image (sum of successive xy sections) (Figure 4a) shows that the bacteria penetrated and invaded the entire collagen matrix to form unique hybrid biomaterials. Interestingly, the magnified top-view confocal image (Figure 4b-c) shows the alignment of bacteria along the collagen scaffold, suggesting the existence of specific recognition of the probiotic by collagen. Similar results were found for col-*La*.

### EXAMPLE 2: Collagen matrix stabilizes probiotics in harsh conditions and improves their metabolic activity

The collagen matrix not merely acts as a hosting scaffold for probiotics, but also provides them with additional protection during storage under harsh conditions (e.g. at 4 °C). Live bacteria excrete metabolites to the media and *lactobacilli*, in particular, excretes lactic acid, which regulates the pH at around 4. We measured the pH evolution to monitor the metabolic activity of the probiotics after storage under harsh conditions (4 °C). The pH evolution of fresh *Lf* and col-*Lf* (without thermal treatment) and after storage at 4 °C during two, four and eight weeks are shown in Figure 5. Col- *Lf* induced a much higher drop of the pH in diluted MRS media in comparison to that of *Lf* (Figure 5a), supporting that probiotics embedded within the collagen matrix retain their metabolic activity and probiotics. Similar behaviors were observed for the col-*La*/*La* pair after two and four weeks of storage at 4 °C. However, col-La stored for eight weeks showed much less activity (Figure 6a).

In addition, we assessed the metabolic activity of stored col-*Lf* and col-*La* at 4 °C using an assay that correlates the reductive capacity of bacteria to their metabolic activity, using an electrochromic polyoxometalate (POM) (González, N. et al. Chem. Commun. 2015, 51). Once reduced, POM exhibits an absorption band in the UV-vis spectrum centered at 820 nm. The evolution of this absorption band after incubating aliquots from the supernatants of col-*Lf* or *Lf* cultures with an aqueous solution of POM was monitored (Figure 5b). The POM reduction activity in the MRS broth media is enhanced when the bacteria is embedded in the collagen matrix in comparison to those of free stored bacteria (Figure 5b). Final values of col-*Lf* stored for two, four and eight weeks were similar to those obtained for fresh samples (stored at room temperature). Similar behavior was observed for the col-*La*/*La* pair after two or eight weeks.

Samples of col-*La* after 2 months of storage showed much less capacity to reduce POM (Figure 6b), in agreement with their inferior ability to reduce pH (*cf*. col-*Lf*) noted above (Figure 6b).

These observations support that the collagen matrix assists in maintaining the core cellular activity of probiotics, especially that of *Lf*, even after their exposure to extended harsh storage conditions. The retention of high metabolic and cellular activities is a critical parameter for the successful use of probiotics in antibiotic-free therapies.

### EXAMPLE 3: Collagen matrix improves the adhesion of probiotics

Collagen is the main structural protein in the extracellular space of various connective tissues in the body. Therefore, another advantage of using collagen as a scaffold for probiotics is the exceptional capacity of this protein to adhere to biological tissues. The adhesion of the probiotic to the vaginal mucosa is of paramount relevance to efficiently treat BV with probiotics. Indeed, if a biomaterial containing highly proliferating probiotics does not adequately adhere to the vaginal mucosa, it would not offer much value for BV therapy.

We evaluated the adhesion properties of col-*Lf*/col-*La* and *Lf*/*La* to endothelial cells using porcine mucin, a material containing heavily glycosylated proteins produced by epithelial tissues, which is a well-established adhesion model. The adherence on immobilized porcine mucin of col-*Lf*/col- *La* and *Lf*/*La* was measured by indirect spectroscopic quantification of adsorbed bacteria labeled with crystal violet (see Experimental Section). As shown in Figure 7, the adhesion of col-*Lf* to mucin was sensibly higher than that of *Lf*, supporting the ability of collagen to enhance the adhesion of entrapped probiotics. The col-*La* formulation also exhibited a higher adhesion to mucin than the *La* probiotic alone (Figure 8). On comparison of the adhesion of col-*Lf* and col-*La* to mucin, a higher signal corresponding to crystal violet was observed for col-*Lf*. This is likely due to the higher CFU value of col-*Lf* in comparison to that of col-*La*, as discussed above.

The typical adhesion model also includes a treatment with bovine serum albumin (BSA) after the immobilization of mucin. BSA blocks the remaining free sites of the protein-sensitive well plates after mucin immobilization. The experiments using BSA (Figure 9) showed no significant difference to that of the corresponding experiments without BSA (Figure 9b). This control experiment allows us to conclude that the increase in absorbance is not due to the potential adsorption of collagen to the protein-biding plates, but is due to the increase of the absorption of the adhered col-*Lf*.

The higher adhesion to mucin of col-probiotics over pristine probiotics evidences the role of the collagen matrix to allow nesting of the entrapped probiotics in a specific tissue, as could be the vaginal mucosa during the pathogenic conditions caused by BV. As stated above, an adequate adhesion of the probiotic-containing material to the vaginal mucosa is crucial to achieve the efficacy of the BV treatment. If the adhesion is not optimal, the probiotics cannot effectively acclimatize and excrete lactic acid in an unfavorable microenvironment and the pathogenic bacteria are likely to remain active, further promoting the infection.

### EXAMPLE 4: col-Lf and col-La for BV therapy

A decisive step to confirm the functionality of col-*Lf* and col-*La* for BV therapy is their capacity to restore the healthy conditions from a simulant BV fluid pH 5.0. As noted from Figure 10, both col- *Lf* and col-*La* were able to shift the pH 5 of the BV medium to that of healthy conditions, pH 4. This pH shift is certainly due to excreted lactic acid by the probiotic bacteria, in agreement with the previous evidences that the bacteria remain alive and active once entrapped into the collagen matrix. Interestingly, the drop in pH with *Lf* and *La* without collagen was much slower and did not reach the healthy pH value of 4.0 (Figure 10).

Two observations are worth highlighting from these results: i) the two materials, col-*Lf* and col-*La*, were capable of shifting the pH from the unhealthy BV medium (pH 5) to the healthy vaginal conditions (pH 4); and ii) the ability to reduce the pH of col-*Lf* or col-La is higher than those of *Lf* or *La*. These observations point out that the collagen matrix stabilizes the probiotics and makes them more robust to withstand the shock of being in a hostile environment, as is the BV fluid. This is of significant interest as it outlines the low efficiency of pristine probiotics as a potential BV treatment, and suggests the need for an additional biocompatible matrix with high adherence properties, such as collagen that stabilizes the probiotics to overcome the hostile BV medium.

## Claims

1. A method for preparing a biomaterial comprising type I collagen, probiotics, and exopolysaccharide wherein the method comprises:
i. contacting a population of probiotic bacteria with collagen monomers,
ii. incubating the mixture obtained in step i) under conditions adequate for the self-assembly of the collagen monomers into collagen fibers, thereby obtaining collagen fibers containing entrapped probiotic bacteria; and
iii. maintaining the collagen fibers containing entrapped probiotic bacteria obtained in step ii) under conditions adequate for the formation of exopolysaccharide (EPS) by the probiotics until the content of EPS in the biomaterial with respect to the collagen content of the biomaterial measured as the sugar content is of at least 2% (w/w).

2. The method according to claim 1 wherein the collagen is type I collagen.

3. The method according to claims 1 or 2 wherein step (ii) is carried out at a pH of between 4 and 9.

4. The method according to any of claims 1 to 3 wherein step ii) is carried out until collagen fibrils are formed which show a diameter of between 1 and 600 nm and/or show a 67 nm periodic staggered D-banding pattern; or wherein, step ii) is performed under continuous stirring.

5. The method according to any of claims 1 to 4 wherein step iii) is carried out by incubating the collagen fibers containing entrapped probiotic in MRS medium; optionally wherein the probiotic is Lactobacillus or Bifidobacterium; optionally wherein the probiotic is selected from *L fermentum, L acidophilus, L crispatus, Ljensenii and, L gasseri. B. breve, B. longum, B. bifidum*, and *B. dentium.*

6. The method according to claim 5, wherein the probiotic is *L fermentum* and/or *L acidophilus* and more particularly *L fermentum* CECT5716 and/or *L acidophilus* CECT903.

7. A biomaterial obtainable by a method of any of claims 1 to 6.

8. A biomaterial comprising a collagen scaffold, probiotics and exopolysaccharide (EPS), wherein the collagen scaffold comprises fibrils between 100 and 500 nm diameter and wherein the sugar content in the biomaterial with respect to the collagen content of the biomaterial is of at least 2% (w/w).

9. The biomaterial according to claim 8 wherein the collagen scaffold comprises fibrils showing a 67 nm periodic staggered D-banding pattern.

10. The biomaterial according to claims 8 or 9 wherein the collagen is type I collagen; optionally wherein the probiotic concentration is of at least 6 ×10¹² CFU per gram of collagen; optionally wherein the EPS is selected from hompolysaccharides (HoPS) and/or a heteropolysaccharide (HepS).

11. The biomaterial according to claim 10, wherein the HoPS is selected from α-glucans, β-glucans and β-fructans, and/or the HePS comprises any of D-glucose, D-galactose, L-rhamnose and N-acetylated monosaccharides selected from N- acetyl-glucosamine (GluNAc), N-acetyl-galactosamine (GalNAc), fucose, glucuronic acid, glycerol and mannose; optionally wherein the EPS is selected from dextran, levan, mutan, alternan, reuteran and inulin-like; optionally wherein the EPS is dextran, levan or a combination thereof.

12. The biomaterial according to any of clams 8 to 11, wherein the probiotic is selected from Lactobacillus and/or Bifidobacterium; optionally wherein the Lactobacillus is selected from *L fermentum, L acidophilus L crispatus, L. jensenii* and, *L gasseri* or wherein the Bifidobacterium is selected from *B. breve, B. longum, B. bifidum,* and *B. dentium* or any combination thereof; optionally wherein the probiotics are *L. fermentum* and/or *L acidophilus* and more particularly, *L fermentum* CECT5716 and/or *L acidophilus* CECT903 or a combination thereof.

13. The biomaterial according to any of claims 8 to 12 for use in medicine.

14. The biomaterial according to any of claims 8 to 12 for use in the treatment of a vaginal infection; optionally wherein the infection is caused by a bacteria, yeast or protozoan parasite; optionally wherein the infection is bacterial vaginosis, trichomonas vaginitis or candidiasis; optionally wherein the infection is bacterial vaginosis; optionally wherein the bacterial vaginosis is caused by overgrowth of normal vagina flora selected from *Gardnerella vaginalis* or *Mobiluncus spp.*

15. A pharmaceutical composition comprising the biomaterial according to any of claims 7 to 14 and a pharmaceutical acceptable excipient suitable for vaginal administration

## Patentansprüche

1. Verfahren zum Herstellen eines Biomaterials umfassend Kollagen Typ I, Probiotika und Exopolysaccharid, wobei das Verfahren Folgendes umfasst:
i. Inkontaktbringen einer Population probiotischer Bakterien mit Kollagenmonomeren,
ii. Inkubieren des in Schritt i) erhaltenen Gemisches unter Bedingungen, die für die Selbstassemblierung der Kollagenmonomere zu Kollagenfasern angemessen sind, wodurch Kollagenfasern erhalten werden, die eingeschlossene probiotische Bakterien enthalten; und
iii. Halten der in Schritt ii) erhaltenen Kollagenfasern, die eingeschlossene probiotische Bakterien enthalten, unter Bedingungen, die für die Bildung von Exopolysaccharid (EPS) durch die Probiotika angemessen sind, bis der Gehalt an EPS im Biomaterial bezogen auf den Kollagengehalt des Biomaterials, gemessen als Zuckergehalt, mindestens 2 Gew.-% beträgt.

2. Verfahren nach Anspruch 1, wobei das Kollagen Kollagen Typ I ist.

3. Verfahren nach Anspruch 1 oder 2, wobei Schritt (ii) bei einem pH zwischen 4 und 9 ausgeführt wird.

4. Verfahren nach einem der Ansprüche 1 bis 3, wobei Schritt ii) ausgeführt wird, bis Kollagenfibrillen gebildet werden, die einen Durchmesser zwischen 1 und 600 nm aufweisen und/oder ein D-Bandenmuster von 67 nm mit periodischer Staffelung zeigen; oder wobei Schritt ii) unter kontinuierlichem Rühren durchgeführt wird.

5. Verfahren nach einem der Ansprüche 1 bis 4, wobei Schritt iii) durch Inkubieren der Kollagenfasern, die eingeschlossenes Probiotikum enthalten, in MRS-Medium ausgeführt wird; wobei optional das Probiotikum Lactobacillus oder Bifidobacterium ist; wobei optional das Probiotikum aus *L fermentum, L acidophilus, L crispatus, L jensenii und L gasseri, B. breve, B. longum, B. bifidum* und *B. dentium* ausgewählt ist.

6. Verfahren nach Anspruch 5, wobei das Probiotikum *L fermentum* und/oder *L acidophilus* und insbesondere *L fermentum* CECT5716 und/oder *L acidophilus* CECT903 ist.

7. Biomaterial, erhältlich durch ein Verfahren nach einem der Ansprüche 1 bis 6.

8. Biomaterial, umfassend ein Kollagengerüst, Probiotika und Exopolysaccharid (EPS), wobei das Kollagengerüst Fibrillen zwischen 100 und 500 nm im Durchmesser umfasst und wobei der Zuckergehalt in dem Biomaterial bezogen auf den Kollagengehalt des Biomaterials mindestens 2 Gew.-% beträgt.

9. Biomaterial nach Anspruch 8, wobei das Kollagengerüst Fibrillen umfasst, die ein D-Bandenmuster von 67 nm mit periodischer Staffelung zeigen.

10. Biomaterial nach Anspruch 8 oder 9, wobei das Kollagen Kollagen Typ I ist; wobei optional die Probiotikum-Konzentration mindestens 6 ×10¹² KBE pro Gramm Kollagen beträgt; wobei optional das EPS aus Hompolysacchariden (HoPS) und/oder einem Heteropolysaccharid (HepS) ausgewählt ist.

11. Biomaterial nach Anspruch 10, wobei das HoPS aus α-Glucanen, β-Glucanen und β-Fructanen ausgewählt ist, und/oder das HePS ein beliebiges von D-Glucose, D-Galactose, L-Rhamnose und N-acetylierten Monosacchariden, ausgewählt aus N-Acetylglucosamin (GluNAc), N-Acetylgalactosamin (GalNAc), Fucose, Glucuronsäure, Glycerin und Mannose ausgewählt ist; wobei optional das EPS aus Dextran, Levan, Mutan, Alternan, Reuteran und inulinartig ausgewählt ist; wobei optional das EPS Dextran, Levan oder eine Kombination davon ist.

12. Biomaterial nach einem der Ansprüche 8 bis 11, wobei das Probiotikum aus Lactobacillus und/oder Bifidobacterium ausgewählt ist; wobei optional das Lactobacillus aus *L fermentum, L acidophilus L crispatus, L. jensenii* und *L gasseri* ausgewählt ist oder wobei das Bifidobacterium aus *B. breve, B. longum, B. bifidum* und *B. dentium* oder einer beliebigen Kombination davon ausgewählt ist; wobei optional die Probiotika *L. fermentum* und/oder *L acidophilus* und insbesondere *L-fermentum* CECT5776 und/oder *L acidophilus* CECT903 oder eine Kombination davon sind.

13. Biomaterial nach einem der Ansprüche 8 bis 12 zur Verwendung in der Medizin.

14. Biomaterial nach einem der Ansprüche 8 bis 12 zur Verwendung bei der Behandlung einer vaginalen Infektion; wobei optional die Infektion durch einen Bakterien-, Hefe- oder Protozoenparasiten hervorgerufen wird; wobei optional die Infektion bakterielle Vaginose, Trichomonas-Vaginitis oder Candidiasis ist; wobei optional die Infektion bakterielle Vaginose ist; wobei optional die bakterielle Vaginose durch Überwuchs von normaler Vaginalflora, ausgewählt aus *Gardnerella vaginalis* oder *Mobiluncus spp,* hervorgerufen wird.

15. Pharmazeutische Zusammensetzung, umfassend das Biomaterial nach einem der Ansprüche 7 bis 14 und einen pharmazeutisch verträglichen Hilfsstoff, der zur vaginalen Verabreichung geeignet ist.

## Revendications

1. Procédé de préparation d'un biomatériau comprenant du collagène de type I, des probiotiques, et un exopolysaccharide, dans lequel le procédé comprend :
i. la mise en contact d'une population de bactéries probiotiques avec des monomères de collagène,
ii. l'incubation du mélange obtenu à l'étape i) dans des conditions adéquates pour l'auto-assemblage des monomères de collagène en fibres de collagène, ce qui permet ainsi d'obtenir des fibres de collagène contenant des bactéries probiotiques piégées ; et
iii. la conservation des fibres de collagène contenant des bactéries probiotiques piégées obtenues à l'étape ii) dans des conditions adéquates pour la formation d'exopolysaccharide (EPS) par les probiotiques jusqu'à ce que la teneur en EPS du biomatériau par rapport à la teneur en collagène du biomatériau mesurée comme étant la teneur en sucre soit d'au moins 2 % (p/p).

2. Procédé selon la revendication 1, dans lequel le collagène est du collagène de type I.

3. Procédé selon les revendications 1 ou 2, dans lequel l'étape (ii) est réalisée à un pH compris entre 4 et 9.

4. Procédé selon l'une quelconque des revendications 1 à 3, dans lequel l'étape ii) est réalisée jusqu'à la formation de fibrilles de collagène présentant un diamètre compris entre 1 et 600 nm et/ou présentant un motif périodique de bandes D étagées de 67 nm ; ou dans lequel l'étape ii) est réalisée sous agitation continue.

5. Procédé selon l'une quelconque des revendications 1 à 4, dans lequel l'étape iii) est réalisée par incubation des fibres de collagène contenant un probiotique piégé en milieu MRS ; éventuellement dans lequel le probiotique est Lactobacillus ou Bifidobacterium ; éventuellement dans lequel le probiotique est choisi parmi *L fermentum, L acidophilus, L crispatus, L jensenii et L gasseri. B. breve, B. longum, B. bifidum,* et *B. dentium.*

6. Procédé selon la revendication 5, dans lequel le probiotique est *L fermentum* et/ou *L acidophilus* et plus particulièrement *L fermentum* CECT5716 et/ou *L acidophilus* CECT903.

7. Biomatériau pouvant être obtenu par un procédé selon l'une quelconque des revendications 1 à 6.

8. Biomatériau comprenant un échafaudage de collagène, des probiotiques et un exopolysaccharide (EPS), dans lequel l'échafaudage de collagène comprend des fibrilles d'un diamètre compris entre 100 et 500 nm et dans lequel la teneur en sucre du biomatériau par rapport à la teneur en collagène du biomatériau est d'au moins 2 % (p/p).

9. Biomatériau selon la revendication 8, dans lequel l'échafaudage de collagène comprend des fibrilles présentant un motif périodique de bandes D étagées de 67 nm.

10. Biomatériau selon les revendications 8 ou 9, dans lequel le collagène est du collagène de type I ; éventuellement dans lequel la concentration en probiotique est d'au moins 6 ×10¹² CFU par gramme de collagène ; éventuellement dans lequel l'EPS est choisi parmi les hompolysaccharides (HoPS) et/ou un hétéropolysaccharide (HepS).

11. Biomatériau selon la revendication 10, dans lequel l'HoPS est choisi parmi les α-glucanes, les β-glucanes et les β-fructanes, et/ou l'HePS comprend l'un quelconque du D-glucose, du D-galactose, du L-rhamnose et des monosaccharides N-acétylés choisis parmi la N-acétyl-glucosamine (GluNAc), la N-acétyl-galactosamine (GalNAc), le fucose, l'acide glucuronique, le glycérol et le mannose ; éventuellement dans lequel l'EPS est choisi parmi le dextrane, le lévane, le mutane, l'alternane, le reutérane et le type inuline ; éventuellement dans lequel l'EPS est du dextrane, du levan ou une combinaison de ceux-ci.

12. Biomatériau selon l'une quelconque des revendications 8 à 11, dans lequel le probiotique est choisi parmi Lactobacillus et/ou Bifidobacterium ; éventuellement dans lequel le Lactobacillus est choisi parmi *L. fermentum, L. acidophilus L crispatus, L. jensenii* et *L. gasseri* ou dans lequel le Bifidobacterium est choisi parmi *B. breve, B. longum, B. bifidum* et *B. dentium* ou une quelconque combinaison de ceux-ci ; éventuellement dans lequel les probiotiques sont *L. fermentum* et/ou *L acidophilus* et plus particulièrement, *L fermentum* CECT5716 et/ou *L acidophilus* CECT903 ou une combinaison de ceux-ci.

13. Biomatériau selon l'une quelconque des revendications 8 à 12 destiné à être utilisé en médecine.

14. Biomatériau selon l'une quelconque des revendications 8 à 12 destiné à être utilisé dans le traitement d'une infection vaginale ; éventuellement dans lequel l'infection est provoquée par une bactérie, une levure ou un parasite protozoaire ; éventuellement dans lequel l'infection est une vaginose bactérienne, une vaginite à trichomonas ou une candidose ; éventuellement dans lequel l'infection est une vaginose bactérienne ; éventuellement dans lequel la vaginose bactérienne est provoquée par une prolifération de la flore vaginale normale choisie parmi *Gardnerella vaginalis* ou *Mobiluncus spp.*

15. Composition pharmaceutique comprenant le biomatériau selon l'une quelconque des revendications 7 à 14 et un excipient pharmaceutique acceptable approprié pour une administration vaginale.
